# EUROPEAN PATENT APPLICATION

(11) **EP 2 333 103 A1**
(43) Date of publication of application: **15.06.2011**
(21) Application number: 09178908.1
(22) Date of filing: 11.12.2009
(51) Int. Cl.: C12Q 1/68

(54) **Differential diagnosis and therapy for kinase inhibitors**

(71) Applicant: Max-Planck-Gesellschaft zur Förderung der Wissenschaften e.V., 80539 München (DE)
(72) Inventor: Ullrich, Axel, 80331 München (DE); Bairlein, Michaela, 81241 München (DE)
(74) Representative: Weiss, Wolfgang

(57) **Abstract**

The present invention relates to novel methods and compositions for prognosing the effectiveness of kinase inhibitors such as sunitinib in the therapy of hyperproliferative and particularly metastatic diseases. Thus, the invention allows the development of individually adapted strategies for treating conditions associated with and/or caused by the occurrence of a given type of hyperproliferating and/or metastatic cells.

SEQUENCE LISTING

<110> Max-Planck-Gesellschaft zur Förderung der Wissenschaften
e.V.

<120> Differential diagnosis and therapy for kinase inhibitors

<130> 45057P EP

<160> 12

<170> PatentIn version 3.3

<210> 1
<211> 2347
<212> PRT
<213> Homo sapiens

<220>
<221> MISC_FEATURE
<222> (1)..(2347)
<223> proto-oncogene c-ros-1 protein precursor

<400> 1

Met Lys Asn Ile Tyr Cys Leu Ile Pro Lys Leu Val Asn Phe Ala Thr
1               5                   10                  15

Leu Gly Cys Leu Trp Ile Ser Val Val Gln Cys Thr Val Leu Asn Ser
            20                  25                  30

Cys Leu Lys Ser Cys Val Thr Asn Leu Gly Gln Gln Leu Asp Leu Gly
        35                  40                  45

Thr Pro His Asn Leu Ser Glu Pro Cys Ile Gln Gly Cys His Phe Trp
    50                  55                  60

Asn Ser Val Asp Gln Lys Asn Cys Ala Leu Lys Cys Arg Glu Ser Cys
65                  70                  75                  80

Glu Val Gly Cys Ser Ser Ala Glu Gly Ala Tyr Glu Glu Glu Val Leu
                85                  90                  95

Glu Asn Ala Asp Leu Pro Thr Ala Pro Phe Ala Ser Ser Ile Gly Ser
            100                 105                 110

His Asn Met Thr Leu Arg Trp Lys Ser Ala Asn Phe Ser Gly Val Lys
        115                 120                 125

Tyr Ile Ile Gln Trp Lys Tyr Ala Gln Leu Leu Gly Ser Trp Thr Tyr
    130                 135                 140

Thr Lys Thr Val Ser Arg Pro Ser Tyr Val Val Lys Pro Leu His Pro
145                 150                 155                 160

Phe Thr Glu Tyr Ile Phe Arg Val Val Trp Ile Phe Thr Ala Gln Leu
                165                 170                 175

Gln Leu Tyr Ser Pro Pro Ser Pro Ser Tyr Arg Thr His Pro His Gly
            180                 185                 190

Val Pro Glu Thr Ala Pro Leu Ile Arg Asn Ile Glu Ser Ser Ser Pro
        195                 200                 205

Asp Thr Val Glu Val Ser Trp Asp Pro Pro Gln Phe Pro Gly Gly Pro
    210                 215                 220

Ile Leu Gly Tyr Asn Leu Arg Leu Ile Ser Lys Asn Gln Lys Leu Asp
225                 230                 235                 240

Ala Gly Thr Gln Arg Thr Ser Phe Gln Phe Tyr Ser Thr Leu Pro Asn
                245                 250                 255

Thr Ile Tyr Arg Phe Ser Ile Ala Ala Val Asn Glu Val Gly Glu Gly
            260                 265                 270

Pro Glu Ala Glu Ser Ser Ile Thr Thr Ser Ser Ser Ala Val Gln Gln
        275                 280                 285

Glu Glu Gln Trp Leu Phe Leu Ser Arg Lys Thr Ser Leu Arg Lys Arg
    290                 295                 300

Ser Leu Lys His Leu Val Asp Glu Ala His Cys Leu Arg Leu Asp Ala
305                 310                 315                 320

Ile Tyr His Asn Ile Thr Gly Ile Ser Val Asp Val His Gln Gln Ile
                325                 330                 335

Val Tyr Phe Ser Glu Gly Thr Leu Ile Trp Ala Lys Lys Ala Ala Asn
            340                 345                 350

Met Ser Asp Val Ser Asp Leu Arg Ile Phe Tyr Arg Gly Ser Gly Leu
        355                 360                 365

Ile Ser Ser Ile Ser Ile Asp Trp Leu Tyr Gln Arg Met Tyr Phe Ile
    370                 375                 380

Met Asp Glu Leu Val Cys Val Cys Asp Leu Glu Asn Cys Ser Asn Ile
385                 390                 395                 400

Glu Glu Ile Thr Pro Pro Ser Ile Ser Ala Pro Gln Lys Ile Val Ala
                405                 410                 415

Asp Ser Tyr Asn Gly Tyr Val Phe Tyr Leu Leu Arg Asp Gly Ile Tyr
            420                 425                 430

Arg Ala Asp Leu Pro Val Pro Ser Gly Arg Cys Ala Glu Ala Val Arg
        435                 440                 445

Ile Val Glu Ser Cys Thr Leu Lys Asp Phe Ala Ile Lys Pro Gln Ala
    450                 455                 460

Lys Arg Ile Ile Tyr Phe Asn Asp Thr Ala Gln Val Phe Met Ser Thr
465                 470                 475                 480

Phe Leu Asp Gly Ser Ala Ser His Leu Ile Leu Pro Arg Ile Pro Phe
                485                 490                 495

Ala Asp Val Lys Ser Phe Ala Cys Glu Asn Asn Asp Phe Leu Val Thr
            500                 505                 510

Asp Gly Lys Val Ile Phe Gln Gln Asp Ala Leu Ser Phe Asn Glu Phe
        515                 520                 525

Ile Val Gly Cys Asp Leu Ser His Ile Glu Glu Phe Gly Phe Gly Asn
    530                 535                 540

Leu Val Ile Phe Gly Ser Ser Ser Gln Leu His Pro Leu Pro Gly Arg
545                 550                 555                 560

Pro Gln Glu Leu Ser Val Leu Phe Gly Ser His Gln Ala Leu Val Gln
                565                 570                 575

Trp Lys Pro Pro Ala Leu Ala Ile Gly Ala Asn Val Ile Leu Ile Ser
            580                 585                 590

Asp Ile Ile Glu Leu Phe Glu Leu Gly Pro Ser Ala Trp Gln Asn Trp
        595                 600                 605

Thr Tyr Glu Val Lys Val Ser Thr Gln Asp Pro Pro Glu Val Thr His
    610                 615                 620

Ile Phe Leu Asn Ile Ser Gly Thr Met Leu Asn Val Pro Glu Leu Gln
625                 630                 635                 640

Ser Ala Met Lys Tyr Lys Val Ser Val Arg Ala Ser Ser Pro Lys Arg
                645                 650                 655

Pro Gly Pro Trp Ser Glu Pro Ser Val Gly Thr Thr Leu Val Pro Ala
            660                 665                 670

Ser Glu Pro Pro Phe Ile Met Ala Val Lys Glu Asp Gly Leu Trp Ser
        675                 680                 685

Lys Pro Leu Asn Ser Phe Gly Pro Gly Glu Phe Leu Ser Ser Asp Ile
    690                 695                 700

Gly Asn Val Ser Asp Met Asp Trp Tyr Asn Asn Ser Leu Tyr Tyr Ser
705                 710                 715                 720

Asp Thr Lys Gly Asp Val Phe Val Trp Leu Leu Asn Gly Thr Asp Ile

                725                 730                 735

Ser Glu Asn Tyr His Leu Pro Ser Ile Ala Gly Ala Gly Ala Leu Ala
            740                 745                 750

Phe Glu Trp Leu Gly His Phe Leu Tyr Trp Ala Gly Lys Thr Tyr Val
        755                 760                 765

Ile Gln Arg Gln Ser Val Leu Thr Gly His Thr Asp Ile Val Thr His
    770                 775                 780

Val Lys Leu Leu Val Asn Asp Met Val Val Asp Ser Val Gly Gly Tyr
785                 790                 795                 800

Leu Tyr Trp Thr Thr Leu Tyr Ser Val Glu Ser Thr Arg Leu Asn Gly
                805                 810                 815

Glu Ser Ser Leu Val Leu Gln Thr Gln Pro Trp Phe Ser Gly Lys Lys
            820                 825                 830

Val Ile Ala Leu Thr Leu Asp Leu Ser Asp Gly Leu Leu Tyr Trp Leu
        835                 840                 845

Val Gln Asp Ser Gln Cys Ile His Leu Tyr Thr Ala Val Leu Arg Gly
    850                 855                 860

Gln Ser Thr Gly Asp Thr Thr Ile Thr Glu Phe Ala Ala Trp Ser Thr
865                 870                 875                 880

Ser Glu Ile Ser Gln Asn Ala Leu Met Tyr Tyr Ser Gly Arg Leu Phe
                885                 890                 895

Trp Ile Asn Gly Phe Arg Ile Ile Thr Thr Gln Glu Ile Gly Gln Lys
            900                 905                 910

Thr Ser Val Ser Val Leu Glu Pro Ala Arg Phe Asn Gln Phe Thr Ile
        915                 920                 925

Ile Gln Thr Ser Leu Lys Pro Leu Pro Gly Asn Phe Ser Phe Thr Pro
    930                 935                 940

Lys Val Ile Pro Asp Ser Val Gln Glu Ser Ser Phe Arg Ile Glu Gly
945                 950                 955                 960

Asn Ala Ser Ser Phe Gln Ile Leu Trp Asn Gly Pro Pro Ala Val Asp
                965                 970                 975

Trp Gly Val Val Phe Tyr Ser Val Glu Phe Ser Ala His Ser Lys Phe
            980                 985                 990

Leu Ala Ser Glu Gln His Ser Leu Pro Val Phe Thr Val Glu Gly Leu
        995                 1000                1005

Glu Pro Tyr Ala Leu Phe Asn Leu Ser Val Thr Pro Tyr Thr Tyr
    1010                1015                1020

Trp Gly Lys Gly Pro Lys Thr Ser Leu Ser Leu Arg Ala Pro Glu
    1025                1030                1035

Thr Val Pro Ser Ala Pro Glu Asn Pro Arg Ile Phe Ile Leu Pro
    1040                1045                1050

Ser Gly Lys Cys Cys Asn Lys Asn Glu Val Val Val Glu Phe Arg
    1055                1060                1065

Trp Asn Lys Pro Lys His Glu Asn Gly Val Leu Thr Lys Phe Glu
    1070                1075                1080

Ile Phe Tyr Asn Ile Ser Asn Gln Ser Ile Thr Asn Lys Thr Cys
    1085                1090                1095

Glu Asp Trp Ile Ala Val Asn Val Thr Pro Ser Val Met Ser Phe
    1100                1105                1110

Gln Leu Glu Gly Met Ser Pro Arg Cys Phe Ile Ala Phe Gln Val
    1115                1120                1125

Arg Ala Phe Thr Ser Lys Gly Pro Gly Pro Tyr Ala Asp Val Val
    1130                1135                1140

Lys Ser Thr Thr Ser Glu Ile Asn Pro Phe Pro His Leu Ile Thr
    1145                1150                1155

Leu Leu Gly Asn Lys Ile Val Phe Leu Asp Met Asp Gln Asn Gln
    1160                1165                1170

Val Val Trp Thr Phe Ser Ala Glu Arg Val Ile Ser Ala Val Cys
    1175                1180                1185

Tyr Thr Ala Asp Asn Glu Met Gly Tyr Tyr Ala Glu Gly Asp Ser
    1190                1195                1200

Leu Phe Leu Leu His Leu His Asn Arg Ser Ser Ser Glu Leu Phe
    1205                1210                1215

Gln Asp Ser Leu Val Phe Asp Ile Thr Val Ile Thr Ile Asp Trp
    1220                1225                1230

Ile Ser Arg His Leu Tyr Phe Ala Leu Lys Glu Ser Gln Asn Gly
    1235                1240                1245

Met Gln Val Phe Asp Val Asp Leu Glu His Lys Val Lys Tyr Pro
    1250                1255                1260

Arg Glu Val Lys Ile His Asn Arg Asn Ser Thr Ile Ile Ser Phe
    1265                1270                1275

Ser Val Tyr Pro Leu Leu Ser Arg Leu Tyr Trp Thr Glu Val Ser
    1280                1285                1290

Asn Phe Gly Tyr Gln Met Phe Tyr Tyr Ser Ile Ile Ser His Thr
    1295                1300                1305

Leu His Arg Ile Leu Gln Pro Thr Ala Thr Asn Gln Gln Asn Lys
    1310                1315                1320

Arg Asn Gln Cys Ser Cys Asn Val Thr Glu Phe Glu Leu Ser Gly
    1325                1330                1335

Ala Met Ala Ile Asp Thr Ser Asn Leu Glu Lys Pro Leu Ile Tyr
    1340                1345                1350

Phe Ala Lys Ala Gln Glu Ile Trp Ala Met Asp Leu Glu Gly Cys
    1355                1360                1365

Gln Cys Trp Arg Val Ile Thr Val Pro Ala Met Leu Ala Gly Lys
    1370                1375                1380

Thr Leu Val Ser Leu Thr Val Asp Gly Asp Leu Ile Tyr Trp Ile
    1385                1390                1395

Ile Thr Ala Lys Asp Ser Thr Gln Ile Tyr Gln Ala Lys Lys Gly
    1400                1405                1410

Asn Gly Ala Ile Val Ser Gln Val Lys Ala Leu Arg Ser Arg His
    1415                1420                1425

Ile Leu Ala Tyr Ser Ser Val Met Gln Pro Phe Pro Asp Lys Ala
    1430                1435                1440

Phe Leu Ser Leu Ala Ser Asp Thr Val Glu Pro Thr Ile Leu Asn
    1445                1450                1455

Ala Thr Asn Thr Ser Leu Thr Ile Arg Leu Pro Leu Ala Lys Thr
    1460                1465                1470

Asn Leu Thr Trp Tyr Gly Ile Thr Ser Pro Thr Pro Thr Tyr Leu
    1475                1480                1485

Val Tyr Tyr Ala Glu Val Asn Asp Arg Lys Asn Ser Ser Asp Leu
    1490                1495                1500

Lys Tyr Arg Ile Leu Glu Phe Gln Asp Ser Ile Ala Leu Ile Glu
    1505                1510                1515

Asp Leu Gln Pro Phe Ser Thr Tyr Met Ile Gln Ile Ala Val Lys
    1520                    1525                    1530

Asn Tyr Tyr Ser Asp Pro Leu Glu His Leu Pro Pro Gly Lys Glu
    1535                    1540                    1545

Ile Trp Gly Lys Thr Lys Asn Gly Val Pro Glu Ala Val Gln Leu
    1550                    1555                    1560

Ile Asn Thr Thr Val Arg Ser Asp Thr Ser Leu Ile Ile Ser Trp
    1565                    1570                    1575

Arg Glu Ser His Lys Pro Asn Gly Pro Lys Glu Ser Val Arg Tyr
    1580                    1585                    1590

Gln Leu Ala Ile Ser His Leu Ala Leu Ile Pro Glu Thr Pro Leu
    1595                    1600                    1605

Arg Gln Ser Glu Phe Pro Asn Gly Arg Leu Thr Leu Leu Val Thr
    1610                    1615                    1620

Arg Leu Ser Gly Gly Asn Ile Tyr Val Leu Lys Val Leu Ala Cys
    1625                    1630                    1635

His Ser Glu Glu Met Trp Cys Thr Glu Ser His Pro Val Thr Val
    1640                    1645                    1650

Glu Met Phe Asn Thr Pro Glu Lys Pro Tyr Ser Leu Val Pro Glu
    1655                    1660                    1665

Asn Thr Ser Leu Gln Phe Asn Trp Lys Ala Pro Leu Asn Val Asn
    1670                    1675                    1680

Leu Ile Arg Phe Trp Val Glu Leu Gln Lys Trp Lys Tyr Asn Glu
    1685                    1690                    1695

Phe Tyr His Val Lys Thr Ser Cys Ser Gln Gly Pro Ala Tyr Val
    1700                    1705                    1710

Cys Asn Ile Thr Asn Leu Gln Pro Tyr Thr Ser Tyr Asn Val Arg
    1715                    1720                    1725

Val Val Val Val Tyr Lys Thr Gly Glu Asn Ser Thr Ser Leu Pro
    1730                    1735                    1740

Glu Ser Phe Lys Thr Lys Ala Gly Val Pro Asn Lys Pro Gly Ile
    1745                    1750                    1755

Pro Lys Leu Leu Glu Gly Ser Lys Asn Ser Ile Gln Trp Glu Lys
    1760                    1765                    1770

Ala Glu Asp Asn Gly Cys Arg Ile Thr Tyr Tyr Ile Leu Glu Ile

1775                 1780                 1785

Arg Lys  Ser Thr Ser Asn Asn  Leu Gln Asn Gln Asn  Leu Arg Trp
    1790                 1795                 1800

Lys Met  Thr Phe Asn Gly Ser  Cys Ser Ser Val Cys  Thr Trp Lys
    1805                 1810                 1815

Ser Lys  Asn Leu Lys Gly Ile  Phe Gln Phe Arg Val  Val Ala Ala
    1820                 1825                 1830

Asn Asn  Leu Gly Phe Gly Glu  Tyr Ser Gly Ile Ser  Glu Asn Ile
    1835                 1840                 1845

Ile Leu  Val Gly Asp Asp Phe  Trp Ile Pro Glu Thr  Ser Phe Ile
    1850                 1855                 1860

Leu Thr  Ile Ile Val Gly Ile  Phe Leu Val Val Thr  Ile Pro Leu
    1865                 1870                 1875

Thr Phe  Val Trp His Arg Arg  Leu Lys Asn Gln Lys  Ser Ala Lys
    1880                 1885                 1890

Glu Gly  Val Thr Val Leu Ile  Asn Glu Asp Lys Glu  Leu Ala Glu
    1895                 1900                 1905

Leu Arg  Gly Leu Ala Ala Gly  Val Gly Leu Ala Asn  Ala Cys Tyr
    1910                 1915                 1920

Ala Ile  His Thr Leu Pro Thr  Gln Glu Glu Ile Glu  Asn Leu Pro
    1925                 1930                 1935

Ala Phe  Pro Arg Glu Lys Leu  Thr Leu Arg Leu Leu  Leu Gly Ser
    1940                 1945                 1950

Gly Ala  Phe Gly Glu Val Tyr  Glu Gly Thr Ala Val  Asp Ile Leu
    1955                 1960                 1965

Gly Val  Gly Ser Gly Glu Ile  Lys Val Ala Val Lys  Thr Leu Lys
    1970                 1975                 1980

Lys Gly  Ser Thr Asp Gln Glu  Lys Ile Glu Phe Leu  Lys Glu Ala
    1985                 1990                 1995

His Leu  Met Ser Lys Phe Asn  His Pro Asn Ile Leu  Lys Gln Leu
    2000                 2005                 2010

Gly Val  Cys Leu Leu Asn Glu  Pro Gln Tyr Ile Ile  Leu Glu Leu
    2015                 2020                 2025

Met Glu  Gly Gly Asp Leu Leu  Thr Tyr Leu Arg Lys  Ala Arg Met
    2030                 2035                 2040

Ala Thr Phe Tyr Gly Pro Leu Leu Thr Leu Val Asp Leu Val Asp
    2045                2050                2055

Leu Cys Val Asp Ile Ser Lys Gly Cys Val Tyr Leu Glu Arg Met
    2060                2065                2070

His Phe Ile His Arg Asp Leu Ala Ala Arg Asn Cys Leu Val Ser
    2075                2080                2085

Val Lys Asp Tyr Thr Ser Pro Arg Ile Val Lys Ile Gly Asp Phe
    2090                2095                2100

Gly Leu Ala Arg Asp Ile Tyr Lys Asn Asp Tyr Tyr Arg Lys Arg
    2105                2110                2115

Gly Glu Gly Leu Leu Pro Val Arg Trp Met Ala Pro Glu Ser Leu
    2120                2125                2130

Met Asp Gly Ile Phe Thr Thr Gln Ser Asp Val Trp Ser Phe Gly
    2135                2140                2145

Ile Leu Ile Trp Glu Ile Leu Thr Leu Gly His Gln Pro Tyr Pro
    2150                2155                2160

Ala His Ser Asn Leu Asp Val Leu Asn Tyr Val Gln Thr Gly Gly
    2165                2170                2175

Arg Leu Glu Pro Pro Arg Asn Cys Pro Asp Asp Leu Trp Asn Leu
    2180                2185                2190

Met Thr Gln Cys Trp Ala Gln Glu Pro Asp Gln Arg Pro Thr Phe
    2195                2200                2205

His Arg Ile Gln Asp Gln Leu Gln Leu Phe Arg Asn Phe Phe Leu
    2210                2215                2220

Asn Ser Ile Tyr Lys Ser Arg Asp Glu Ala Asn Asn Ser Gly Val
    2225                2230                2235

Ile Asn Glu Ser Phe Glu Gly Glu Asp Gly Asp Val Ile Cys Leu
    2240                2245                2250

Asn Ser Asp Asp Ile Met Pro Val Ala Leu Met Glu Thr Lys Asn
    2255                2260                2265

Arg Glu Gly Leu Asn Tyr Met Val Leu Ala Thr Glu Cys Gly Gln
    2270                2275                2280

Gly Glu Glu Lys Ser Glu Gly Pro Leu Gly Ser Gln Glu Ser Glu
    2285                2290                2295

Ser Cys Gly Leu Arg Lys Glu Glu Lys Glu Pro His Ala Asp Lys
    2300                    2305                    2310

Asp Phe Cys Gln Glu Lys Gln Val Ala Tyr Cys Pro Ser Gly Lys
    2315                    2320                    2325

Pro Glu Gly Leu Asn Tyr Ala Cys Leu Thr His Ser Gly Tyr Gly
    2330                    2335                    2340

Asp Gly Ser Asp
    2345

<210>  2
<211>  1161
<212>  PRT
<213>  Homo sapiens

<220>
<221>  MISC_FEATURE
<222>  (1)..(1161)
<223>  BMP-2 inducible kinase isoform a

<400>  2
   Met Lys Lys Phe Ser Arg Met Pro Lys Ser Glu Gly Gly Ser Gly Gly
   1               5                   10                  15
   Gly Ala Ala Gly Gly Gly Ala Gly Gly Ala Gly Ala Gly Ala Gly Cys
               20                  25                  30
   Gly Ser Gly Gly Ser Ser Val Gly Val Arg Val Phe Ala Val Gly Arg
           35                  40                  45
   His Gln Val Thr Leu Glu Glu Ser Leu Ala Glu Gly Gly Phe Ser Thr
       50                  55                  60
   Val Phe Leu Val Arg Thr His Gly Gly Ile Arg Cys Ala Leu Lys Arg
   65                  70                  75                  80
   Met Tyr Val Asn Asn Met Pro Asp Leu Asn Val Cys Lys Arg Glu Ile
                   85                  90                  95
   Thr Ile Met Lys Glu Leu Ser Gly His Lys Asn Ile Val Gly Tyr Leu
               100                 105                 110
   Asp Cys Ala Val Asn Ser Ile Ser Asp Asn Val Trp Glu Val Leu Ile
           115                 120                 125
   Leu Met Glu Tyr Cys Arg Ala Gly Gln Val Val Asn Gln Met Asn Lys
       130                 135                 140
   Lys Leu Gln Thr Gly Phe Thr Glu Pro Glu Val Leu Gln Ile Phe Cys
   145                 150                 155                 160
   Asp Thr Cys Glu Ala Val Ala Arg Leu His Gln Cys Lys Thr Pro Ile
                   165                 170                 175
   Ile His Arg Asp Leu Lys Val Glu Asn Ile Leu Leu Asn Asp Gly Gly
               180                 185                 190
   Asn Tyr Val Leu Cys Asp Phe Gly Ser Ala Thr Asn Lys Phe Leu Asn
           195                 200                 205
   Pro Gln Lys Asp Gly Val Asn Val Val Glu Glu Glu Ile Lys Lys Tyr
       210                 215                 220
   Thr Thr Leu Ser Tyr Arg Ala Pro Glu Met Ile Asn Leu Tyr Gly Gly
   225                 230                 235                 240
   Lys Pro Ile Thr Thr Lys Ala Asp Ile Trp Ala Leu Gly Cys Leu Leu
                   245                 250                 255
   Tyr Lys Leu Cys Phe Phe Thr Leu Pro Phe Gly Glu Ser Gln Val Ala
               260                 265                 270
   Ile Cys Asp Gly Asn Phe Thr Ile Pro Asp Asn Ser Arg Tyr Ser Arg
           275                 280                 285
   Asn Ile His Cys Leu Ile Arg Phe Met Leu Glu Pro Asp Pro Glu His
       290                 295                 300
   Arg Pro Asp Ile Phe Gln Val Ser Tyr Phe Ala Phe Lys Phe Ala Lys
   305                 310                 315                 320
   Lys Asp Cys Pro Val Ser Asn Ile Asn Asn Ser Ser Ile Pro Ser Ala
                   325                 330                 335
   Leu Pro Glu Pro Met Thr Ala Ser Glu Ala Ala Ala Arg Lys Ser Gln
               340                 345                 350
   Ile Lys Ala Arg Ile Thr Asp Thr Ile Gly Pro Thr Glu Thr Ser Ile
           355                 360                 365
   Ala Pro Arg Gln Arg Pro Lys Ala Asn Ser Ala Thr Thr Ala Thr Pro
       370                 375                 380
   Ser Val Leu Thr Ile Gln Ser Ser Ala Thr Pro Val Lys Val Leu Ala
   385                 390                 395                 400
   Pro Gly Glu Phe Gly Asn His Arg Pro Lys Gly Ala Leu Arg Pro Gly
                   405                 410                 415
   Asn Gly Pro Glu Ile Leu Leu Gly Gln Gly Pro Pro Gln Gln Pro Pro
               420                 425                 430
   Gln Gln His Arg Val Leu Gln Gln Leu Gln Gln Gly Asp Trp Arg Leu
           435                 440                 445
   Gln Gln Leu His Leu Gln His Arg His Pro His Gln Gln Gln Gln Gln
       450                 455                 460
   Gln Gln Gln Gln Gln Gln Gln Gln Gln Gln Gln Gln Gln Gln Gln Gln
   465                 470                 475                 480
   Gln Gln Gln Gln Gln Gln His His His His His His His His Leu Leu
                   485                 490                 495
   Gln Asp Ala Tyr Met Gln Gln Tyr Gln His Ala Thr Gln Gln Gln Gln
               500                 505                 510
   Met Leu Gln Gln Gln Phe Leu Met His Ser Val Tyr Gln Pro Gln Pro
           515                 520                 525
   Ser Ala Ser Gln Tyr Pro Thr Met Met Pro Gln Tyr Gln Gln Ala Phe
       530                 535                 540
   Phe Gln Gln Gln Met Leu Ala Gln His Gln Pro Ser Gln Gln Gln Ala
   545                 550                 555                 560
   Ser Pro Glu Tyr Leu Thr Ser Pro Gln Glu Phe Ser Pro Ala Leu Val
                   565                 570                 575
   Ser Tyr Thr Ser Ser Leu Pro Ala Gln Val Gly Thr Ile Met Asp Ser
               580                 585                 590
   Ser Tyr Ser Ala Asn Arg Ser Val Ala Asp Lys Glu Ala Ile Ala Asn
           595                 600                 605
   Phe Thr Asn Gln Lys Asn Ile Ser Asn Pro Pro Asp Met Ser Gly Trp
       610                 615                 620
   Asn Pro Phe Gly Glu Asp Asn Phe Ser Lys Leu Thr Glu Glu Glu Leu
   625                 630                 635                 640
   Leu Asp Arg Glu Phe Asp Leu Leu Arg Ser Asn Arg Leu Glu Glu Arg
                   645                 650                 655
   Ala Ser Ser Asp Lys Asn Val Asp Ser Leu Ser Ala Pro His Asn His
               660                 665                 670
   Pro Pro Glu Asp Pro Phe Gly Ser Val Pro Phe Ile Ser His Ser Gly
           675                 680                 685
   Ser Pro Glu Lys Lys Ala Glu His Ser Ser Ile Asn Gln Glu Asn Gly
       690                 695                 700
   Thr Ala Asn Pro Ile Lys Asn Gly Lys Thr Ser Pro Ala Ser Lys Asp
   705                 710                 715                 720
   Gln Arg Thr Gly Lys Lys Thr Ser Val Gln Gly Gln Val Gln Lys Gly
                   725                 730                 735
   Asn Asp Glu Ser Glu Ser Asp Phe Glu Ser Asp Pro Pro Ser Pro Lys
               740                 745                 750
   Ser Ser Glu Glu Glu Glu Gln Asp Asp Glu Glu Val Leu Gln Gly Glu
           755                 760                 765
   Gln Gly Asp Phe Asn Asp Asp Asp Thr Glu Pro Glu Asn Leu Gly His
       770                 775                 780
   Arg Pro Leu Leu Met Asp Ser Glu Asp Glu Glu Glu Glu Glu Lys His
   785                 790                 795                 800
   Ser Ser Asp Ser Asp Tyr Glu Gln Ala Lys Ala Lys Tyr Ser Asp Met
                   805                 810                 815
   Ser Ser Val Tyr Arg Asp Arg Ser Gly Ser Gly Pro Thr Gln Asp Leu
               820                 825                 830
   Asn Thr Ile Leu Leu Thr Ser Ala Gln Leu Ser Ser Asp Val Ala Val
           835                 840                 845
   Glu Thr Pro Lys Gln Glu Phe Asp Val Phe Gly Ala Val Pro Phe Phe
       850                 855                 860
   Ala Val Arg Ala Gln Gln Pro Gln Gln Glu Lys Asn Glu Lys Asn Leu
   865                 870                 875                 880
   Pro Gln His Arg Phe Pro Ala Ala Gly Leu Glu Gln Glu Glu Phe Asp
                   885                 890                 895
   Val Phe Thr Lys Ala Pro Phe Ser Lys Lys Val Asn Val Gln Glu Cys
               900                 905                 910
   His Ala Val Gly Pro Glu Ala His Thr Ile Pro Gly Tyr Pro Lys Ser
           915                 920                 925
   Val Asp Val Phe Gly Ser Thr Pro Phe Gln Pro Phe Leu Thr Ser Thr
       930                 935                 940
   Ser Lys Ser Glu Ser Asn Glu Asp Leu Phe Gly Leu Val Pro Phe Asp
   945                 950                 955                 960
   Glu Ile Thr Gly Ser Gln Gln Gln Lys Val Lys Gln Arg Ser Leu Gln
                   965                 970                 975
   Lys Leu Ser Ser Arg Gln Arg Arg Thr Lys Gln Asp Met Ser Lys Ser
               980                 985                 990
   Asn Gly Lys Arg His His Gly Thr Pro Thr Ser Thr Lys Lys Thr Leu
           995                 1000                1005
   Lys Pro Thr Tyr Arg Thr Pro Glu Arg Ala Arg Arg His Lys Lys
       1010                1015                1020
   Val Gly Arg Arg Asp Ser Gln Ser Ser Asn Glu Phe Leu Thr Ile
       1025                1030                1035
   Ser Asp Ser Lys Glu Asn Ile Ser Val Ala Leu Thr Asp Gly Lys
       1040                1045                1050
   Asp Arg Gly Asn Val Leu Gln Pro Glu Glu Ser Leu Leu Asp Pro
       1055                1060                1065
   Phe Gly Ala Lys Pro Phe His Ser Pro Asp Leu Ser Trp His Pro
       1070                1075                1080
   Pro His Gln Gly Leu Ser Asp Ile Arg Ala Asp His Asn Thr Val
       1085                1090                1095
   Leu Pro Gly Arg Pro Arg Gln Asn Ser Leu His Gly Ser Phe His
       1100                1105                1110
   Ser Ala Asp Val Leu Lys Met Asp Asp Phe Gly Ala Val Pro Phe
       1115                1120                1125
   Thr Glu Leu Val Val Gln Ser Ile Thr Pro His Gln Ser Gln Gln
       1130                1135                1140
   Ser Gln Pro Val Glu Leu Asp Pro Phe Gly Ala Ala Pro Phe Pro
       1145                1150                1155
   Ser Lys Gln
       1160

<210> 3
<211> 662
<212> PRT
<213> Homo sapiens

<220>
<221> MISC_FEATURE
<222> (1)..(662)
<223> BMP-2 inducible kinase isoform b

<400> 3

Met Lys Lys Phe Ser Arg Met Pro Lys Ser Glu Gly Gly Ser Gly Gly
1               5                   10                  15

Gly Ala Ala Gly Gly Gly Ala Gly Gly Ala Gly Ala Gly Ala Gly Cys
            20                  25                  30

Gly Ser Gly Gly Ser Ser Val Gly Val Arg Val Phe Ala Val Gly Arg
        35                  40                  45

His Gln Val Thr Leu Glu Glu Ser Leu Ala Glu Gly Gly Phe Ser Thr
    50                  55                  60

Val Phe Leu Val Arg Thr His Gly Gly Ile Arg Cys Ala Leu Lys Arg
65                  70                  75                  80

Met Tyr Val Asn Asn Met Pro Asp Leu Asn Val Cys Lys Arg Glu Ile
                85                  90                  95

Thr Ile Met Lys Glu Leu Ser Gly His Lys Asn Ile Val Gly Tyr Leu
            100                 105                 110

Asp Cys Ala Val Asn Ser Ile Ser Asp Asn Val Trp Glu Val Leu Ile
        115                 120                 125

Leu Met Glu Tyr Cys Arg Ala Gly Gln Val Val Asn Gln Met Asn Lys
    130                 135                 140

Lys Leu Gln Thr Gly Phe Thr Glu Pro Glu Val Leu Gln Ile Phe Cys
145                 150                 155                 160

Asp Thr Cys Glu Ala Val Ala Arg Leu His Gln Cys Lys Thr Pro Ile
                165                 170                 175

Ile His Arg Asp Leu Lys Val Glu Asn Ile Leu Leu Asn Asp Gly Gly
            180                 185                 190

Asn Tyr Val Leu Cys Asp Phe Gly Ser Ala Thr Asn Lys Phe Leu Asn
        195                 200                 205

Pro Gln Lys Asp Gly Val Asn Val Val Glu Glu Glu Ile Lys Lys Tyr
    210                 215                 220

Thr Thr Leu Ser Tyr Arg Ala Pro Glu Met Ile Asn Leu Tyr Gly Gly
225                 230                 235                 240

Lys Pro Ile Thr Thr Lys Ala Asp Ile Trp Ala Leu Gly Cys Leu Leu
                245                 250                 255

Tyr Lys Leu Cys Phe Phe Thr Leu Pro Phe Gly Glu Ser Gln Val Ala
            260                 265                 270

Ile Cys Asp Gly Asn Phe Thr Ile Pro Asp Asn Ser Arg Tyr Ser Arg
        275                 280                 285

Asn Ile His Cys Leu Ile Arg Phe Met Leu Glu Pro Asp Pro Glu His
    290                 295                 300

Arg Pro Asp Ile Phe Gln Val Ser Tyr Phe Ala Phe Lys Phe Ala Lys
305                 310                 315                 320

Lys Asp Cys Pro Val Ser Asn Ile Asn Asn Ser Ser Ile Pro Ser Ala
                325                 330                 335

Leu Pro Glu Pro Met Thr Ala Ser Glu Ala Ala Ala Arg Lys Ser Gln
            340                 345                 350

Ile Lys Ala Arg Ile Thr Asp Thr Ile Gly Pro Thr Glu Thr Ser Ile
        355                 360                 365

Ala Pro Arg Gln Arg Pro Lys Ala Asn Ser Ala Thr Thr Ala Thr Pro
    370                 375                 380

Ser Val Leu Thr Ile Gln Ser Ser Ala Thr Pro Val Lys Val Leu Ala
385                 390                 395                 400

Pro Gly Glu Phe Gly Asn His Arg Pro Lys Gly Ala Leu Arg Pro Gly
                405                 410                 415

Asn Gly Pro Glu Ile Leu Leu Gly Gln Gly Pro Pro Gln Gln Pro Pro
            420                 425                 430

Gln Gln His Arg Val Leu Gln Gln Leu Gln Gln Gly Asp Trp Arg Leu
        435                 440                 445

Gln Gln Leu His Leu Gln His Arg His Pro His Gln Gln Gln Gln Gln
    450                 455                 460

Gln Gln Gln Gln Gln Gln Gln Gln Gln Gln Gln Gln Gln Gln Gln Gln
465                 470                 475                 480

Gln Gln Gln Gln Gln Gln His His His His His His His His Leu Leu
                485                 490                 495

Gln Asp Ala Tyr Met Gln Gln Tyr Gln His Ala Thr Gln Gln Gln Gln
            500                 505                 510

Met Leu Gln Gln Gln Phe Leu Met His Ser Val Tyr Gln Pro Gln Pro
        515                 520                 525

Ser Ala Ser Gln Tyr Pro Thr Met Met Pro Gln Tyr Gln Gln Ala Phe
    530                 535                 540

Phe Gln Gln Gln Met Leu Ala Gln His Gln Pro Ser Gln Gln Gln Ala
545                 550                 555                 560

Ser Pro Glu Tyr Leu Thr Ser Pro Gln Glu Phe Ser Pro Ala Leu Val
                565                 570                 575

Ser Tyr Thr Ser Ser Leu Pro Ala Gln Val Gly Thr Ile Met Asp Ser
            580                 585                 590

Ser Tyr Ser Ala Asn Arg Ser Val Ala Asp Lys Glu Ala Ile Ala Asn
        595                 600                 605

Phe Thr Asn Gln Lys Asn Ile Ser Asn Pro Pro Asp Met Ser Gly Trp
    610                 615                 620

Asn Pro Phe Gly Glu Asp Asn Phe Ser Lys Leu Thr Glu Glu Glu Leu
625                 630                 635                 640

Leu Asp Arg Glu Phe Asp Leu Leu Arg Ser Ser Lys Gly His Leu Lys
            645                 650                 655

Ala Tyr Phe Ala Ser Gln
            660

<210>  4
<211>  979
<212>  PRT
<213>  Homo sapiens

<220>
<221>  MISC_FEATURE
<222>  (1)..(979)
<223>  NIMA-related kinase 9

<400>  4

<210>  5
<211>  187
<212>  PRT
<213>  Homo sapiens

<220>
<221>  MISC_FEATURE
<222>  (1)..(187)
<223>  NME4: nucleoside diphosphate kinase 4 precursor

<400>  5

Met Gly Gly Leu Phe Trp Arg Ser Ala Leu Arg Gly Leu Arg Cys Gly
1               5                   10                  15

Pro Arg Ala Pro Gly Pro Ser Leu Leu Val Arg His Gly Ser Gly Gly
            20                  25                  30

Pro Ser Trp Thr Arg Glu Arg Thr Leu Val Ala Val Lys Pro Asp Gly
        35                  40                  45

Val Gln Arg Arg Leu Val Gly Asp Val Ile Gln Arg Phe Glu Arg Arg
    50                  55                  60

Gly Phe Thr Leu Val Gly Met Lys Met Leu Gln Ala Pro Glu Ser Val
65                  70                  75                  80

Leu Ala Glu His Tyr Gln Asp Leu Arg Arg Lys Pro Phe Tyr Pro Ala
                85                  90                  95

Leu Ile Arg Tyr Met Ser Ser Gly Pro Val Val Ala Met Val Trp Glu
            100                 105                 110

Gly Tyr Asn Val Val Arg Ala Ser Arg Ala Met Ile Gly His Thr Asp
        115                 120                 125

Ser Ala Glu Ala Ala Pro Gly Thr Ile Arg Gly Asp Phe Ser Val His
    130                 135                 140

Ile Ser Arg Asn Val Ile His Ala Ser Asp Ser Val Glu Gly Ala Gln
145                 150                 155                 160

Arg Glu Ile Gln Leu Trp Phe Gln Ser Ser Glu Leu Val Ser Trp Ala
                165                 170                 175

Asp Gly Gly Gln His Ser Ser Ile His Pro Ala
            180                 185

<210>  6
<211>  729
<212>  PRT
<213>  Homo sapiens

<220>
<221>  MISC_FEATURE
<222>  (1)..(729)
<223>  TBK1: TANK-binding kinase 1

<400>  6

<210>  7
<211>  7368
<212>  DNA
<213>  Homo sapiens

<220>
<221>  mRNA
<222>  (1)..(7368)

<223>  c-ros oncogene 1 , receptor tyrosine kinase (ROS1)

<400>  7
caagctttca agcattcaaa ggtctaaatg aaaaaggcta agtattattt caaaaggcaa      60

gtatatccta atatagcaaa acaaacaaag caaaatccat cagctactcc tccaattgaa     120

gtgatgaagc ccaaataatt catatagcaa aatggagaaa attagaccgg ccatctaaaa     180

atctgccatt ggtgaagtga tgaagaacat ttactgtctt attccgaagc ttgtcaattt     240

tgcaactctt ggctgcctat ggatttctgt ggtgcagtgt acagttttaa atagctgcct     300

aaagtcgtgt gtaactaatc tgggccagca gcttgacctt ggcacaccac ataatctgag     360

tgaaccgtgt atccaaggat gtcacttttg gaactctgta gatcagaaaa actgtgcttt     420

aaagtgtcgg gagtcgtgtg aggttggctg tagcagcgcg gaaggtgcat atgaagagga     480

agtactggaa aatgcagacc taccaactgc tccctttgct tcttccattg gaagccacaa     540

tatgacatta cgatggaaat ctgcaaactt ctctggagta aaatacatca ttcagtggaa     600

atatgcacaa cttctgggaa gctggactta tactaagact gtgtccagac cgtcctatgt     660

ggtcaagccc ctgcacccct tcactgagta cattttccga gtggtttgga tcttcacagc     720

gcagctgcag ctctactccc ctccaagtcc cagttacagg actcatcctc atggagttcc     780

tgaaactgca cctttgatta ggaatattga gagctcaagt cccgacactg tggaagtcag     840

ctgggatcca cctcaattcc caggtggacc tattttgggt tataacttaa ggctgatcag     900

caaaaatcaa aaattagatg cagggacaca gagaaccagt ttccagtttt actccacttt     960

accaaatact atctacaggt tttctattgc agcagtaaat gaagttggtg agggtccaga    1020

agcagaatct agtattacca cttcatcttc agcagttcaa caagaggaac agtggctctt    1080

tttatccaga aaaacttctc taagaaagag atctttaaaa catttagtag atgaagcaca    1140

ttgccttcgg ttggatgcta tataccataa tattacagga atatctgttg atgtccacca    1200

gcaaattgtt tatttctctg aaggaactct catatgggcg aagaaggctg ccaacatgtc    1260

tgatgtatct gacctgagaa tttttacag aggttcagga ttaatttctt ctatctccat    1320

agattggctt tatcaaagaa tgtatttcat catggatgaa ctggtatgtg tctgtgattt    1380

agagaactgc tcaaacatcg aggaaattac tccaccctct attagtgcac ctcaaaaaat    1440

tgtggctgat tcatacaatg ggtatgtctt ttacctcctg agagatggca tttatagagc    1500

agaccttcct gtaccatctg gccggtgtgc agaagctgtg cgtattgtgg agagttgcac    1560

gttaaaggac tttgcaatca agccacaagc caagcgaatc atttacttca atgacactgc    1620

ccaagtcttc atgtcaacat ttctggatgg ctctgcttcc catctcatcc tacctcgcat    1680

cccctttgct gatgtgaaaa gttttgcttg tgaaaacaat gactttcttg tcacagatgg    1740

caaggtcatt ttccaacagg atgctttgtc ttttaatgaa ttcatcgtgg gatgtgacct    1800

gagtcacata gaagaatttg ggtttggtaa cttggtcatc tttggctcat cctcccagct    1860

gcaccctctg ccaggccgcc cgcaggagct ttcggtgctg tttggctctc accaggctct    1920

tgttcaatgg aagcctcctg cccttgccat aggagccaat gtcatcctga tcagtgatat    1980

tattgaactc tttgaattag gcccttctgc ctggcagaac tggacctatg aggtgaaagt 2040
atccacccaa gaccctcctg aagtcactca tattttcttg aacataagtg gaaccatgct 2100
gaatgtacct gagctgcaga gtgctatgaa atacaaggtt tctgtgagag caagttctcc 2160
aaagaggcca ggcccctggt cagagccctc agtgggtact accctggtgc cagctagtga 2220
accaccattt atcatggctg tgaaagaaga tgggctttgg agtaaaccat taaatagctt 2280
tggcccagga gagttcttat cctctgatat aggaaatgtg tcagacatgg attggtataa 2340
caacagcctc tactacagtg acacgaaagg cgacgttttt gtgtggctgc tgaatgggac 2400
ggatatctca gagaattatc acctacccag cattgcagga gcaggggctt tagcttttga 2460
gtggctgggt cactttctct actgggctgg aaagacatat gtgatacaaa ggcagtctgt 2520
gttgacggga cacacagaca ttgttaccca cgtgaagcta ttggtgaatg acatggtggt 2580
ggattcagtt ggtggatatc tctactggac cacactctat tcagtggaaa gcaccagact 2640
aaatggggaa agttcccttg tactacagac acagccttgg ttttctggga aaaaggtaat 2700
tgctctaact ttagacctca gtgatgggct cctgtattgg ttggttcaag acagtcaatg 2760
tattcacctg tacacagctg ttcttcgggg acagagcact ggggatacca ccatcacaga 2820
atttgcagcc tggagtactt ctgaaatttc ccagaatgca ctgatgtact atagtggtcg 2880
gctgttctgg atcaatggct ttaggattat cacaactcaa gaaataggtc agaaaaccag 2940
tgtctctgtt ttggaaccag ccagatttaa tcagttcaca attattcaga catcccttaa 3000
gcccctgcca gggaactttt cctttacccc taaggttatt ccagattctg ttcaagagtc 3060
ttcatttagg attgaaggaa atgcttcaag ttttcaaatc ctgtggaatg gtccccctgc 3120
ggtagactgg ggtgtagttt tctacagtgt agaatttagt gctcattcta agttcttggc 3180
tagtgaacaa cactctttac ctgtatttac tgtggaagga ctggaacctt atgccttatt 3240
taatctttct gtcactcctt atacctactg gggaaagggc cccaaaacat ctctgtcact 3300
tcgagcacct gaaacagttc catcagcacc agagaacccc agaatattta tattaccaag 3360
tggaaaatgc tgcaacaaga atgaagttgt ggtggaattt aggtggaaca aacctaagca 3420
tgaaaatggg gtgttaacaa aatttgaaat tttctacaat atatccaatc aaagtattac 3480
aaacaaaaca tgtgaagact ggattgctgt caatgtcact ccctcagtga tgtcttttca 3540
acttgaaggc atgagtccca gatgctttat tgccttccag gttagggcct ttacatctaa 3600
ggggccagga ccatatgctg acgttgtaaa gtctacaaca tcagaaatca acccatttcc 3660
tcacctcata actcttcttg gtaacaagat agttttttta gatatggatc aaaatcaagt 3720
tgtgtggacg ttttcagcag aaagagttat cagtgccgtt tgctacacag ctgataatga 3780
gatgggatat tatgctgaag gggactcact ctttcttctg cacttgcaca atcgctctag 3840
ctctgagctt ttccaagatt cactggtttt tgatatcaca gttattacaa ttgactggat 3900
ttcaaggcac ctctactttg cactgaaaga atcacaaaat ggaatgcaag tatttgatgt 3960
tgatcttgaa cacaaggtga aatatcccag agaggtgaag attcacaata ggaattcaac 4020
aataatttct ttttctgtat atcctctttt aagtcgcttg tattggacag aagtttccaa 4080

ttttggctac cagatgttct actacagtat tatcagtcac accttgcacc gaattctgca 4140
acccacagct acaaaccaac aaaacaaaag gaatcaatgt tcttgtaatg tgactgaatt 4200
tgagttaagt ggagcaatgg ctattgatac ctctaaccta gagaaaccat tgatatactt 4260
tgccaaagca caagagatct gggcaatgga tctggaaggc tgtcagtgtt ggagagttat 4320
cacagtacct gctatgctcg caggaaaaac ccttgttagc ttaactgtgg atggagatct 4380
tatatactgg atcatcacag caaaggacag cacacagatt tatcaggcaa agaaaggaaa 4440
tggggccatc gtttcccagg tgaaggccct aaggagtagg catatcttgg cttacagttc 4500
agttatgcag ccttttccag ataaagcgtt tctgtctcta gcttcagaca ctgtggaacc 4560
aactatactt aatgccacta acactagcct cacaatcaga ttacctctgg ccaagacaaa 4620
cctcacatgg tatggcatca ccagccctac tccaacatac ctggtttatt atgcagaagt 4680
taatgacagg aaaaacagct ctgacttgaa atatagaatt ctggaatttc aggacagtat 4740
agctcttatt gaagatttac aaccattttc aacatacatg atacagatag ctgtaaaaaa 4800
ttattattca gatcctttgg aacatttacc accaggaaaa gagatttggg gaaaaactaa 4860
aaatggagta ccagaggcag tgcagctcat taatacaact gtgcggtcag acaccagcct 4920
cattatatct tggagagaat ctcacaagcc aaatggacct aaagaatcag tccgttatca 4980
gttggcaatc tcacacctgg ccctaattcc tgaaactcct ctaagacaaa gtgaatttcc 5040
aaatggaagg ctcactctcc ttgttactag actgtctggt ggaaatattt atgtgttaaa 5100
ggttcttgcc tgccactctg aggaaatgtg gtgtacagag agtcatcctg tcactgtgga 5160
aatgtttaac acaccagaga aaccttattc cttggttcca gagaacacta gtttgcaatt 5220
taattggaag gctccattga atgttaacct catcagattt tgggttgagc tacagaagtg 5280
gaaatacaat gagttttacc atgttaaaac ttcatgcagc caaggtcctg cttatgtctg 5340
taatatcaca aatctacaac cttatacttc atataatgtc agagtagtgg tggtttataa 5400
gacgggagaa aatagcacct cacttccaga aagctttaag acaaaagctg gagtcccaaa 5460
taaaccaggc attcccaaat tactagaagg gagtaaaaat tcaatacagt gggagaaagc 5520
tgaagataat ggatgtagaa ttacatacta tatccttgag ataagaaaga gcacttcaaa 5580
taatttacag aaccagaatt taaggtggaa gatgacattt aatggatcct gcagtagtgt 5640
ttgcacatgg aagtccaaaa acctgaaagg aatatttcag ttcagagtag tagctgcaaa 5700
taatctaggg tttggtgaat atagtggaat cagtgagaat attatattag ttggagatga 5760
tttttggata ccagaaacaa gtttcatact tactattata gttggaatat ttctggttgt 5820
tacaatccca ctgacctttg tctggcatag aagattaaag aatcaaaaaa gtgccaagga 5880
aggggtgaca gtgcttataa acgaagacaa agagttggct gagctgcgag gtctggcagc 5940
cggagtaggc ctggctaatg cctgctatgc aatacatact cttccaaccc aagaggagat 6000
tgaaaatctt cctgccttcc ctcgggaaaa actgactctg cgtctcttgc tgggaagtgg 6060
agcctttgga gaagtgtatg aaggaacagc agtggacatc ttaggagttg gaagtggaga 6120

aatcaaagta gcagtgaaga ctttgaagaa gggttccaca gaccaggaga agattgaatt 6180

cctgaaggag gcacatctga tgagcaaatt taatcatccc aacattctga agcagcttgg 6240

agtttgtctg ctgaatgaac cccaatacat tatcctggaa ctgatggagg gaggagacct 6300

tcttacttat ttgcgtaaag cccggatggc aacgttttat ggtcctttac tcaccttggt 6360

tgaccttgta gacctgtgtg tagatatttc aaaaggctgt gtctacttgg aacggatgca 6420

tttcattcac agggatctgg cagctagaaa ttgccttgtt tccgtgaaag actataccag 6480

tccacggata gtgaagattg gagactttgg actcgccaga gacatctata aaaatgatta 6540

ctatagaaag agaggggaag gcctgctccc agttcggtgg atggctccag aaagtttgat 6600

ggatggaatc ttcactactc aatctgatgt atggtctttt ggaattctga tttgggagat 6660

tttaactctt ggtcatcagc cttatccagc tcattccaac cttgatgtgt taaactatgt 6720

gcaaacagga gggagactgg agccaccaag aaattgtcct gatgatctgt ggaatttaat 6780

gacccagtgc tgggctcaag aacccgacca aagacctact tttcatagaa ttcaggacca 6840

acttcagtta ttcagaaatt ttttcttaaa tagcatttat aagtccagag atgaagcaaa 6900

caacagtgga gtcataaatg aaagctttga aggtgaagat ggcgatgtga tttgtttgaa 6960

ttcagatgac attatgccag ttgctttaat ggaaacgaag aaccgagaag ggttaaacta 7020

tatggtactt gctacagaat gtggccaagg tgaagaaaag tctgagggtc ctctaggctc 7080

ccaggaatct gaatcttgtg gtctgaggaa agaagagaag gaaccacatg cagacaaaga 7140

tttctgccaa gaaaaacaag tggcttactg cccttctggc aagcctgaag gcctgaacta 7200

tgcctgtctc actcacagtg gatatggaga tgggtctgat taatagcgtt gtttgggaaa 7260

tagagagttg agataaacac tctcattcag tagttactga aagaaaactc tgctagaatg 7320

ataaatgtca tggtggtcta taactccaaa taaacaatgc aacgttcc 7368

<210> 8
<211> 3859
<212> DNA
<213> Homo sapiens

<220>
<221> mRNA
<222> (1)..(3859)
<223> BMP2 inducible kinase (BMP2K), transcript variant 1

<400> 8
cggggcccag gctgtgcgct tggggagcgc ggaatgtgag gcttggcggg ccgcagcacg 60

ctcggacggg ccaggggcgg cgacccctcg cggacgcccg gctgcgcgcc gggccgggga 120

cttgcccttg cacgctccct gcgccctcca gctcgccggc gggaccatga agaagttctc 180

tcggatgccc aagtcggagg gcggcagcgg cggcggagcg gcgggtggcg gggctggcgg 240

ggccggggcc ggggccggct gcggctccgg cggctcgtcc gtgggggtcc gggtgttcgc 300

ggtcggccgc caccaggtca ccctggaaga gtcgctggcc gaaggtggat tctccacagt 360

tttcctcgtg cgtactcacg gtggaatccg atgtgcattg aagcgaatgt atgtcaataa 420

catgccagac ctcaatgttt gtaaaaggga aattacaatt atgaaagagc tatctggtca 480
caaaaatatt gtgggctatt tggactgtgc tgttaattca attagtgata atgtatggga 540
agtccttatc ttaatggaat attgtcgagc tggacaggta gtgaatcaaa tgaataagaa 600
gctacagacg ggttttacag aaccagaagt gttacagata ttctgtgata cctgtgaagc 660
tgttgcaagg ttgcatcagt gtaagactcc aataattcac cgggatctga aggtagaaaa 720
tattttgttg aatgatggtg ggaactatgt actttgtgac tttggcagtg ccactaataa 780
atttcttaat cctcaaaaag atggagttaa tgtagtagaa gaagaaatta aaaagtatac 840
aactctgtca tacagagccc ctgaaatgat caacctttat ggagggaaac ccatcaccac 900
caaggctgat atctgggcac tgggatgtct actctataaa ctttgtttct tcactcttcc 960
ttttggtgag agtcaggttg ctatctgtga tggcaacttc accatcccag acaattctcg 1020
ttactcccgt aacatacatt gcttaataag gttcatgctt gaaccagatc cggaacatag 1080
acctgatata tttcaagtgt catattttgc atttaaattt gccaaaaagg attgtccagt 1140
ctccaacatc aataattctt ctattccttc agctcttcct gaaccgatga ctgctagtga 1200
agcagctgct aggaaaagcc aaataaaagc cagaataaca gataccattg gaccaacaga 1260
aacctcaatt gcaccaagac aaagaccaaa ggccaactct gctactactg ccactcccag 1320
tgtgctgacc attcaaagtt cagcaacacc tgttaaagtc cttgctcctg gtgaattcgg 1380
taaccataga ccaaaagggg cactaagacc tggaaatggc cctgaaattt tattgggtca 1440
gggacctcct cagcagccgc cacagcagca tagagtactc cagcaactac agcagggaga 1500
ttggagatta cagcaactcc atttacagca tcgtcatcct caccagcagc agcagcagca 1560
gcagcagcaa cagcaacagc agcagcagca acagcaacag cagcagcagc agcagcagca 1620
gcagcaccac caccaccacc accaccacct acttcaagat gcttatatgc agcagtatca 1680
acatgcaaca cagcagcaac agatgcttca acaacaattt ttaatgcatt cggtatatca 1740
accacaacct tctgcatcac agtatcctac aatgatgccg cagtatcagc aggctttctt 1800
tcaacagcag atgctagctc aacatcagcc gtctcaacaa caggcatcac ctgaatatct 1860
tacctcccct caagagttct caccagcctt agtttcctac acttcatcac ttccagctca 1920
ggttggaacc ataatggact cctcctatag tgccaatagg tcagttgctg ataaagaggc 1980
cattgcaaat ttcacaaatc agaagaacat cagcaatcca cctgatatgt cagggtggaa 2040
tccttttgga gaggataatt tctctaagtt aacagaagag gaactattgg acagagaatt 2100
tgaccttcta agatcaaata ggctcgagga gagagcatcc tcagataaga atgtagactc 2160
actttctgct ccacataacc atcctccaga agatcctttt ggttctgttc ctttcatttc 2220
tcattcaggt tctcctgaaa agaaagctga acattcatct ataaatcaag aaaatggcac 2280
tgcaaaccct atcaagaacg gtaaaacaag tccagcatct aaagatcagc ggactggaaa 2340
gaaaacctca gtacagggtc aagtgcaaaa ggggaatgat gaatctgaaa gtgattttga 2400
atcagatccc ccttctccta agagcagtga agaggaagag caagatgatg aagaagttct 2460
tcagggggaa caaggagatt ttaatgatga tgatactgaa ccagaaaatc tgggtcatag 2520

gcctctcctc atggattctg aagatgagga agaagaggag aaacatagct ctgattctga 2580

ttatgagcag gctaaagcaa agtacagtga catgagctct gtctacagag acagatctgg 2640

cagtggacca acccaagatc ttaatacaat actcctcacc tcagcccaat tatcctctga 2700

tgttgcagtg gagactccca aacaggagtt tgatgtattt ggcgctgtcc ccttctttgc 2760

agtgcgtgct caacagcccc agcaagaaaa gaatgaaaag aacctccctc aacacaggtt 2820

tcctgctgca ggactggagc aggaggaatt tgatgtattc acaaaggcgc cttttagcaa 2880

gaaggtgaat gtacaagaat gccatgcagt ggggcctgag gcacatacta tccctggtta 2940

tcccaaaagt gtagatgtat ttggctccac tccatttcag cccttcctca catcaacaag 3000

taaaagtgaa agcaatgagg acctttttgg gcttgtgccc tttgatgaaa taacggggag 3060

ccagcagcaa aaagtcaaac agcgcagctt acagaaactg tcctctcgcc aaaggcgcac 3120

aaagcaggat atgtccaaaa gtaatgggaa gcggcatcat ggcacgccaa ctagcacaaa 3180

gaagactttg aagcctacct atcgcactcc agagagggct cgcaggcaca aaaaagtggg 3240

ccgccgagac tctcaaagta gcaatgaatt tttaaccatc tcagactcca aggagaacat 3300

tagtgttgca ctgactgatg ggaaagatag ggggaatgtc ttacaacctg aggagagcct 3360

gttggacccc ttcggtgcca agcccttcca ttctccagac ctgtcatggc accctccaca 3420

tcagggcctg agcgacatcc gtgctgatca caatactgtc ctgccagggc ggccaagaca 3480

aaattcacta catgggtcat tccatagtgc agatgtattg aaaatggatg attttggtgc 3540

cgtgcccttt acagaacttg tggtgcaaag catcactcca catcagtccc aacagtccca 3600

accagtcgaa ttagacccat ttggtgctgc tccatttcct tctaaacagt agatacttct 3660

gatggattct cggcattaac tcctgtttca aaaaagtgtg aacagtttta tgaatttgaa 3720

agaaaatttg gtagctcttt atagcattca ttcttaaaga tcagtcagaa taggtgattt 3780

ctaaataaac caaatagaag aatgaaaaaa aaaaaaaaaa aaaaaaaaaa aaaaaaaaaa 3840

aaaaaaaaaa aaaaaaaaa 3859

<210> 9
<211> 2681
<212> DNA
<213> Homo sapiens

<220>
<221> mRNA
<222> (1)..(2681)
<223> BMP2 inducible kinase (BMP2K), transcript variant 2

<400> 9
cggggcccag gctgtgcgct tggggagcgc ggaatgtgag gcttggcggg ccgcagcacg 60

ctcggacggg ccaggggcgg cgacccctcg cggacgcccg gctgcgcgcc gggccgggga 120

cttgcccttg cacgctccct gcgccctcca gctcgccggc gggaccatga agaagttctc 180

tcggatgccc aagtcggagg gcggcagcgg cggcggagcg gcgggtggcg gggctggcgg 240

ggccggggcc ggggccggct gcggctccgg cggctcgtcc gtgggggtcc gggtgttcgc 300

ggtcggccgc caccaggtca ccctggaaga gtcgctggcc gaaggtggat tctccacagt 360
tttcctcgtg cgtactcacg gtggaatccg atgtgcattg aagcgaatgt atgtcaataa 420
catgccagac ctcaatgttt gtaaaaggga aattacaatt atgaaagagc tatctggtca 480
caaaaatatt gtgggctatt tggactgtgc tgttaattca attagtgata atgtatggga 540
agtccttatc ttaatggaat attgtcgagc tggacaggta gtgaatcaaa tgaataagaa 600
gctacagacg ggttttacag aaccagaagt gttacagata ttctgtgata cctgtgaagc 660
tgttgcaagg ttgcatcagt gtaagactcc aataattcac cgggatctga aggtagaaaa 720
tattttgttg aatgatggtg ggaactatgt actttgtgac tttggcagtg ccactaataa 780
atttcttaat cctcaaaaag atggagttaa tgtagtagaa gaagaaatta aaaagtatac 840
aactctgtca tacagagccc ctgaaatgat caacctttat ggagggaaac ccatcaccac 900
caaggctgat atctgggcac tgggatgtct actctataaa ctttgtttct tcactcttcc 960
ttttggtgag agtcaggttg ctatctgtga tggcaacttc accatcccag acaattctcg 1020
ttactcccgt aacatacatt gcttaataag gttcatgctt gaaccagatc cggaacatag 1080
acctgatata tttcaagtgt catattttgc atttaaattt gccaaaaagg attgtccagt 1140
ctccaacatc aataattctt ctattccttc agctcttcct gaaccgatga ctgctagtga 1200
agcagctgct aggaaaagcc aaataaaagc cagaataaca gataccattg gaccaacaga 1260
aacctcaatt gcaccaagac aaagaccaaa ggccaactct gctactactg ccactcccag 1320
tgtgctgacc attcaaagtt cagcaacacc tgttaaagtc cttgctcctg gtgaattcgg 1380
taaccataga ccaaaagggg cactaagacc tggaaatggc cctgaaattt tattgggtca 1440
gggacctcct cagcagccgc cacagcagca tagagtactc cagcaactac agcagggaga 1500
ttggagatta cagcaactcc atttacagca tcgtcatcct caccagcagc agcagcagca 1560
gcagcagcaa cagcaacagc agcagcagca acagcaacag cagcagcagc agcagcagca 1620
gcagcaccac caccaccacc accaccacct acttcaagat gcttatatgc agcagtatca 1680
acatgcaaca cagcagcaac agatgcttca acaacaattt ttaatgcatt cggtatatca 1740
accacaacct tctgcatcac agtatcctac aatgatgccg cagtatcagc aggctttctt 1800
tcaacagcag atgctagctc aacatcagcc gtctcaacaa caggcatcac ctgaatatct 1860
tacctcccct caagagttct caccagcctt agtttcctac acttcatcac ttccagctca 1920
ggttggaacc ataatggact cctcctatag tgccaatagg tcagttgctg ataaagaggc 1980
cattgcaaat ttcacaaatc agaagaacat cagcaatcca cctgatatgt cagggtggaa 2040
tccttttgga gaggataatt tctctaagtt aacagaagag gaactattgg acagagaatt 2100
tgaccttcta agatcaagta agggacactt gaaggcttat tttgcttcac agtaaaataa 2160
cagctctatt attattcagc aaggccaaag acttttgaga atgtgtatgg aaaattcttt 2220
ttgtgcattt gagggcaaaa ttcaggccat cttcttatac atatactatc aaattatgtt 2280
gtgtgcatta gaaatcagtt gcttgatagt agctattaaa cccaatattg ctgatagtat 2340

gctaatatcc taaaacttaa atattgcata tctatgaatg ttaaattcag aatatctcta 2400

aaacatggaa aattgatgtt tcaataaaaa gggagacatt ttattatttt gccttactaa 2460

tgattttgca gctctgtttt tctgcacact caatagaaaa tattgtgggt ctgagatgcc 2520

ctttgaaaat gcctgaaaga aaacatgggc tactacatta tgttaatgtt ttgtaatgtc 2580

cttttaatga gtggtgacat aaaagggctg ctttgttgtc ctgatatggc aaaaattaaa 2640

taaattcttt ttcactttaa atcagaaaaa aaaaaaaaaa a 2681

<210> 10
<211> 5560
<212> DNA
<213> Homo sapiens

<220>
<221> mRNA
<222> (1)..(5560)
<223> NIMA (never in mitosis gene a)- related kinase 9 (NEK9)

<400> 10
ctgtttccgg gtcgggggc aggagccacc atcgagcttc gcggggttgc tgggctgacg 60

gatccgcggg ccggcatctg aagcgagcgg gacgcagcgc ggccagggcc tccgggcata 120

cgcaggctgg tccccaaggc ccgcggccgc cgccatgtcg gtgctgggcg agtacgagcg 180

acactgcgat tccatcaact cggactttgg gagcgagtcc gggggttgcg gggactcgag 240

tccggggcct agcgccagtc aggggccgcg agccggcggc ggcgcggcgg agcaggagga 300

actgcactac atccccatcc gcgtcctggg ccgcggcgcc ttcggggaag ccacgctgta 360

ccgccgcacc gaggatgact cactggttgt gtggaaggaa gtcgatttga cccggctgtc 420

tgagaaggaa cgtcgtgatg ccttgaatga gattgttatt ctggcactgc tgcagcacga 480

caacattatt gcctactaca atcacttcat ggacaatacc acgctgctga ttgagctgga 540

atattgtaat ggagggaacc tgtatgacaa aatccttcgt cagaaggaca agttgtttga 600

ggaagagatg gtggtgtggt acctatttca gattgtttca gcagtgagct gcatccataa 660

agctggaatc cttcatagag atataaagac attaaatatt tttctgacca aggcaaacct 720

gataaaactt ggagattatg gcctagcaaa gaaacttaat tctgagtatt ccatggctga 780

gacgcttgtg ggaaccccat attacatgtc tccagagctc tgtcaaggag taaagtacaa 840

tttcaagtct gatatctggg cagttggctg cgtcattttt gaactgctta ccttaaagag 900

gacgtttgat gctacaaacc cacttaacct gtgtgtgaag atcgtgcaag gaattcgggc 960

catggaagtt gactctagcc agtactcttt ggaattgatc caaatggttc attcgtgcct 1020

tgaccaggat cctgagcaga gacctactgc agatgaactt ctagatcgcc ctcttctcag 1080

gaaacgcagg agagagatgg aggaaaaagt cactctgctt aatgcaccta caaagagacc 1140

aaggtcaagc actgtgactg aagcacccat tgctgtagta acatcacgaa ccagtgaagt 1200

ctatgtttgg ggtggtggaa aatccacccc ccagaaactg gatgttatca agagtggctg 1260

tagtgcccgg caggtctgtg cagggaatac ccactttgct gtggtcacag tggagaagga 1320

actgtacact tgggtgaaca tgcaaggagg cactaaactc catggtcagc tgggccatgg 1380

agacaaagcc tcctatcgac agccaaagca tgtggaaaag ttgcaaggca aagctatccg 1440

tcaggtgtca tgtggtgatg atttcactgt ctgtgtgact gatgagggtc agctctatgc 1500

cttcggatca gattattatg gctgcatggg ggtggacaaa gttgctggcc ctgaagtgct 1560

agaacccatg cagctgaact tcttcctcag caatccagtg gagcaggtct cctgtggaga 1620

taatcatgtg gtggttctga cacgaaacaa ggaagtctat tcttggggct gtggcgaata 1680

tggacgactg ggtttggatt cagaagagga ttattataca ccacaaaagg tggatgttcc 1740

caaggccttg attattgttg cagttcaatg tggctgtgat gggacatttc tgttgaccca 1800

gtcaggcaaa gtgctggcct gtggactcaa tgaattcaat aagctgggtc tgaatcagtg 1860

catgtcggga attatcaacc atgaagcata ccatgaagtt ccctacacaa cgtcctttac 1920

cttggccaaa cagttgtcct tttataagat ccgtaccatt gccccaggca agactcacac 1980

agctgctatt gatgagcgag gccggctgct gacctttggc tgcaacaagt gtgggcagct 2040

gggcgttggg aactacaaga agcgtctggg aatcaacctg ttggggggac cccttggtgg 2100

gaagcaagtg atcagggtct cctgcggtga tgagtttacc attgctgcca ctgatgataa 2160

tcacattttt gcctggggca atggtggtaa tggccgcctg gcaatgaccc ccacagagag 2220

accacatggc tctgatatct gtacctcatg gcctcggcct atttttggat ctctgcatca 2280

tgtcccggac ctgtcttgcc gtggatggca taccattctc atcgttgaga aagtattgaa 2340

ttctaagacc atccgttcca atagcagtgg cttatccatt ggaactgtgt ttcagagctc 2400

tagcccggga ggaggcggcg ggggcggcgg tggtgaagaa gaggacagtc agcaggaatc 2460

tgaaactcct gacccaagtg gaggcttccg aggaacaatg gaagcagacc gaggaatgga 2520

aggtttaatc agtcccacag aggccatggg gaacagtaat ggggccagca gctcctgtcc 2580

tggctggctt cgaaaggagc tggaaaatgc agaatttatc cccatgcctg acagcccatc 2640

tcctctcagt gcagcgtttt cagaatctga gaaagatacc ctgccctatg aagagctgca 2700

aggactcaaa gtggcctctg aagctccttt ggaacacaaa ccccaagtag aagcctcgtc 2760

acctcggctg aatcctgcag taacctgtgc tgggaaggga acaccactga ctcctcctgc 2820

gtgtgcgtgc agctctctgc aggtggaggt tgagagattg cagggtctgg tgttaaagtg 2880

tctggctgaa caacagaagc tacagcaaga aaacctccag atttttaccc aactgcagaa 2940

gttgaacaag aaattagaag gagggcagca ggtggggatg cattccaaag gaactcagac 3000

agcaaaggaa gagatggaaa tggatccaaa gcctgactta gattcagatt cctggtgcct 3060

cctgggaaca gactcctgta gacccagcct ctagtctcct gagcctatag agcccccagg 3120

agactgggac ccaaagaact tcacagcaca cttaccgaat gcagagagca gctttcctgg 3180

ctttgttcac ttgcagaaaa ggagcgcaag gcagaggctc tgaagcactt tccttgtaca 3240

tttggagagt ggcattgcct tttagatagg atctaggagt gattttattg ttttggagaa 3300

tggaagggcc cccatggccc tggctttgtc atcagtgact gccatagcaa cagcagctct 3360

gtacctcatc tgttgatccc acctttgaag aggagacaca gtgctcacct taattgcgct 3420

ggtagcagct tatatcccat gtatcatttt caccattgat tggaagctgc cttgggaatt 3480
cagtaccagg cattacccct ctgggtggga gagggagaag tgtaaagttg gagtgggctg 3540
gaatcaggtg tggcccgccc agtgtcctct gcagagtggt gaagtagtct ggccctcttg 3600
ggagccctga gtccaggaaa atatgtctga tggagtcaat ctagggcttg tttcgaaaaa 3660
gttcagttac tctgtgcagc taaatgcttt aggaggaaag gtaggcttag gttgcttttc 3720
ctctgagggt tgattgaaat ttcttcagtg aggaatagag aaagggcagg accctcctca 3780
tcacacagct ggtgtttcag gctgtgacca atgcagggtg ggatttccta actgtggatg 3840
aggggatgag gtgtctctga gggatgaggt gtctcagaga attgagtcca tggggcagtc 3900
agaatagcct taagagaaaa tcatgaagga gaagaggtcc tcctttagct gcctctactt 3960
ggtatcttag agagggctta gagggctctc agtcttctgc ccatgaaaag acttctttga 4020
gcctctgcct tcatggctct tagggttctg atcttgatat cagcagcccc aaccactttc 4080
tttctgaatg tctagtcagt atttttcccc ttttggtgtt ttatgaagcc atgtggtaac 4140
gaatgaatct gtatcatttt tcctacctga gtggcccaaa gccagcacca aaccctggga 4200
gtccctgaag cctaacagaa caggtagaac ttgcaaaagg aatttggctg agagctcact 4260
tctaatcctg tactcactgt gtctttgtag atagaaacaa gctagcttta ccaaagagaa 4320
atagtgtcat agaagaacaa aacttcatat agaaagttct aggcaagtat ttgatggttt 4380
ccttaaggat gtgagctttg tatttccacc tagccttgta aaatgttcct gtggtatttt 4440
gtgtcacaca tcctaccttt gatgagtctc acatcccacc ttcctataaa cagctaaatt 4500
aattttgttt catcttcccc agaccaaaat gtttgataat cttatcaaca ttgtgggagg 4560
tcttgtgcaa tggaaatttt gccatttctc caaaactggt ggcataaagg ctgatgcttg 4620
gggagaaccc cattgctcgg gacaggcaac tctgttcaat gggatcttct ttggtttgat 4680
gttcccattg ttttctcagt tctgggaagc ctagtacatt agtactaatg taatcactga 4740
aaccttttct tgaaataagg gaagcagcca aactttgatt aaagttgcaa gttctgggga 4800
cttgcggggg ttgtcataaa ctgtaacagt gggttttggt tcagcatgta aatgcaactt 4860
tgattttctt gaggaccgat tgacctgtca tgtccctgta tcctcatgct catcatctca 4920
gcaggcctga gaggctgggt cagtttgggt gttcatcatg aggattgctt ctgccatgga 4980
gctgatggac atgggcaggt tgctgagaag gtggggtgaa agtgagtgcc gggggtgggt 5040
gagtgccctg gtcttgttca taggggagcc tttccctagc agtggaacgc tgtggtcatt 5100
ttctctagca tattcccttg ggaagtctag atttgctatt aatctggctg agaatctaag 5160
ttctgtgcct tagagacagt ttgcactttc ccatattgtg cctgggacag ccatatgatt 5220
ttttttccca ccaaacaagt atgcaaacag aaaccagttc aaagggggat ggagtaaaag 5280
atgaggcagt agaaatgcct ttgaatggtt ttctgtagct aattctcttt aaattttgtc 5340
ctgctttttt tctttatgca gtgctaggtg ttttaagttt tctagtagta ttgcttttga 5400
gttacagtat aacctgagtt actcctctgc tctaacattg ttgcagaaga actagtttat 5460

tgttagccag gttgcttgaa aggttgagag tggagtggtt tggcatttct gttttaaata 5520

aacatttaag ctcttaatca gtaaaaaaaa aaaaaaaaaa 5560

<210> 11
<211> 1059
<212> DNA
<213> Homo sapiens

<220>
<221> mRNA
<222> (1)..(1059)
<223> NME4: non-metastatic cells 4, protein expressed in

<400> 11
cgcgctcaca gcggcccgcg ggccgggcgt catgggcggc ctcttctggc gctccgcgct 60

gcgggggctg cgctgcggcc cgcgggcccc gggcccgagc ctgctagtgc gccacggctc 120

gggagggccc tcctggaccc gggagcggac cctggtggcg gtgaagcccg atggcgtgca 180

acggcggctc gttggggacg tgatccagcg ctttgagagg cggggcttca cgctggtggg 240

gatgaagatg ctgcaggcac cagagagcgt ccttgccgag cactaccagg acctgcggag 300

gaagcccttc taccctgccc tcatccgcta catgagctct gggcctgtgg tggccatggt 360

ctgggaaggg tacaatgtcg tccgcgcctc gagggccatg attggacaca ccgactcggc 420

tgaggctgcc ccaggaacca taaggggtga cttcagcgtc cacatcagca ggaatgtcat 480

ccacgccagc gactccgtgg aggggcccca gcgggagatc cagctgtggt tccagagcag 540

tgagctggtg agctgggcag acgggggcca gcacagcagc atccacccag cctgaggctc 600

aagctgccct taccacccca tcccccacgc aggaccaact acctccgtca gcaagaaccc 660

aagcccacat ccaaacctgc ctgtcccaaa ccacttactt ccctgttcac ctctgcccca 720

ccccagccca gaggagtttg agccaccaac ttcagtgcct ttctgtaccc caagccagca 780

caagattgga ccaatccttt ttgcaccaaa gtgccggaca acctttgtgg tggggggggg 840

tcttcacatt atcataacct ctcctctaaa ggggaggcat taaaattcac tgtgcccagc 900

acatgggtgg tacactaatt atgacttccc ccagctctga ggtagaaatg acgcctttat 960

gcaagttgta aggagttgaa cagtaaagag gaagttttgc acacccaaaa aaaaaaaaaa 1020

aaaaaaaaaa aaaaaaaaaa aaaaaaaaaa aaaaaaaaa 1059

<210> 12
<211> 2982
<212> DNA
<213> Homo sapiens

<220>
<221> mRNA
<222> (1)..(2982)
<223> TBK1: TANK-binding kinase 1

<400> 12
gccggcggtg gcgcggcgga gacccggctg gtataacaag aggattgcct gatccagcca 60

agatgcagag cacttctaat catctgtggc ttttatctga tattttaggc caaggagcta 120

ctgcaaatgt ctttcgtgga agacataaga aaactggtga tttatttgct atcaaagtat 180
ttaataacat aagcttcctt cgtccagtgg atgttcaaat gagagaattt gaagtgttga 240
aaaaactcaa tcacaaaaat attgtcaaat tatttgctat tgaagaggag acaacaacaa 300
gacataaagt acttattatg gaattttgtc catgtgggag tttatacact gttttagaag 360
aaccttctaa tgcctatgga ctaccagaat ctgaattctt aattgttttg cgagatgtgg 420
tgggtggaat gaatcatcta cgagagaatg gtatagtgca ccgtgatatc aagccaggaa 480
atatcatgcg tgttataggg gaagatggac agtctgtgta caaactcaca gattttggtg 540
cagctagaga attagaagat gatgagcagt ttgtttctct gtatggcaca gaagaatatt 600
tgcaccctga tatgtatgag agagcagtgc taagaaaaga tcatcagaag aaatatggag 660
caacagttga tctttggagc attggggtaa cattttacca tgcagctact ggatcactgc 720
catttagacc ctttgaaggg cctcgtagga ataaagaagt gatgtataaa ataattacag 780
gaaagccttc tggtgcaata tctggagtac agaaagcaga aaatggacca attgactgga 840
gtggagacat gcctgtttct tgcagtcttt ctcggggtct tcaggttcta cttacccctg 900
ttcttgcaaa catccttgaa gcagatcagg aaaagtgttg gggttttgac cagttttttg 960
cagaaactag tgatatactt caccgaatgg taattcatgt tttttcgcta caacaaatga 1020
cagctcataa gatttatatt catagctata atactgctac tatatttcat gaactggtat 1080
ataaacaaac caaaattatt tcttcaaatc aagaacttat ctacgaaggg cgacgcttag 1140
tcttagaacc tggaaggctg gcacaacatt tccctaaaac tactgaggaa aaccctatat 1200
ttgtagtaag ccgggaacct ctgaatacca taggattaat atatgaaaaa atttccctcc 1260
ctaaagtaca tccacgttat gatttagacg gggatgctag catggctaag gcaataacag 1320
gggttgtgtg ttatgcctgc agaattgcca gtaccttact gctttatcag gaattaatgc 1380
gaaaggggat acgatggctg attgaattaa ttaaagatga ttacaatgaa actgttcaca 1440
aaaagacaga agttgtgatc acattggatt tctgtatcag aaacattgaa aaaactgtga 1500
aagtatatga aaagttgatg aagatcaacc tggaagcggc agagttaggt gaaatttcag 1560
acatacacac caaattgttg agactttcca gttctcaggg aacaatagaa accagtcttc 1620
aggatatcga cagcagatta tctccaggtg gatcactggc agacgcatgg gcacatcaag 1680
aaggcactca tccgaaagac agaaatgtag aaaaactaca agtcctgtta aattgcatga 1740
cagagattta ctatcagttc aaaaaagaca aagcagaacg tagattagct tataatgaag 1800
aacaaatcca caaatttgat aagcaaaaac tgtattacca tgccacaaaa gctatgacgc 1860
actttacaga tgaatgtgtt aaaaagtatg aggcattttt gaataagtca gaagaatgga 1920
taagaaagat gcttcatctt aggaaacagt tattatcgct gactaatcag tgttttgata 1980
ttgaagaaga agtatcaaaa tatcaagaat atactaatga gttacaagaa actctgcctc 2040
agaaaatgtt tacagcttcc agtggaatca aacataccat gaccccaatt tatccaagtt 2100
ctaacacatt agtagaaatg actcttggta tgaagaaatt aaaggaagag atggaagggg 2160

tggttaaaga acttgctgaa aataaccaca ttttagaaag gtttggctct ttaaccatgg 2220

atggtggcct tcgcaacgtt gactgtcttt agctttctaa tagaagttta agaaaagttt 2280

ccgtttgcac aagaaaataa cgcttgggca ttaaatgaat gcctttatag atagtcactt 2340

gtttctacaa ttcagtattt gatgtggtcg tgtaaatatg tacaatattg taaatacata 2400

aaaaatatac aaatttttgg ctgctgtgaa gatgtaattt tatcttttaa catttataat 2460

tatatgagga aatttgacct cagtgatcac gagaagaaag ccatgaccga ccaatatgtt 2520

gacatactga tcctctactc tgagtggggc taaataagtt attttctctg accgcctact 2580

ggaaatattt ttaagtggaa ccaaaatagg catccttaca aatcaggaag actgacttga 2640

cacgtttgta aatggtagaa cggtggctac tgtgagtggg gagcagaacc gcaccactgt 2700

tatactggga taacaatttt tttgagaagg ataaagtggc attattttat tttacaaggt 2760

gcccagatcc cagttatcct tgtatccatg taatttcaga tgaattatta agcaaacatt 2820

ttaaagtgaa ttcattatta aaaactattc atttttttcc tttggccata aatgtgtaat 2880

tgtcattaaa attctaaggt catttcaact gttttaagct gtatatttct ttaattctgc 2940

ttactatttc atggaaaaaa ataaatttct caattttaat gt 2982

## Description

The present invention relates to novel methods and compositions for prognosing the effectiveness of kinase inhibitors such as sunitinib in the therapy of hyperproliferative and particularly metastatic diseases. Thus, the invention allows the development of individually adapted strategies for treating conditions associated with and/or caused by the occurrence of a given type of hyperproliferating and/or metastatic cells.

Medical research has found several more or less efficient drugs and methods for diagnosing, treating and preventing hyperproliferative diseases. A major problem in the treatment of hyperproliferative diseases is the large variety of diseases and conditions resulting from or being caused by the occurrence of hyperproliferating cells in a multicellular organism. The variety of hyperproliferating diseases both results from the multiplicity of various molecular causes and trigger events, and from the multiplicity of different cell types existing in an organism such as a mammal. For treating a specific diagnosed hyperproliferative disease such as a specific cancer disease having a specific molecular profile, consequently not any drug and not any method having antiproliferative effects can be used. As a consequence, the diagnosis and treatment of hyperproliferative diseases requires on the one hand the development of new medicaments having specific antiproliferative effects and differential methods of treatment. On the other hand, an attending physician has to decide or predict which of the available drugs and methods of treatment may be successfully applied for the treatment or prevention of a specific diagnosed hyperproliferative disease.

Due to the diversity of hyperproliferative diseases, generally agents are used for their therapy or prevention, which modulate mechanisms being equally changed in a lot of hyperproliferative cells and thus are more or less effective in the treatment of various hyperproliferative diseases or conditions. However, in some cases such an approach is not suitable for treating of a subject suffering from a specific kind of a hyperproliferative disease.

Protein kinases, e.g tyrosine kinases, are part of intracellular signal cascades which regulate cell division, differentiation and/or apoptosis and thus are involved directly or indirectly in the control of cell proliferation. For example, cell receptor associated tyrosine kinases are involved in the transmission of growth signals from receptors on the cell surface to the nucleus, where the cell division is started. These signal cascades are often disturbed in hyperproliferating cells such as cancer cells, for example due to mutations in the sequence of tyrosine kinase encoding genes involved in the cell cycle regulation. Compared to the corresponding wildtype cells such hyperproliferating cells may exhibit a decreased or increased level of one or more tyrosine kinases. Therefore, genes encoding tyrosine kinases which are involved in the intracellular transmission of growth signals and the expression of which is altered in hyperproliferating cells, belong to the group of proto-oncogenes.

The human gene ROS1 (V-ros UR2 sarcoma virus oncogene homolog 1) is a proto-oncogene, highly expressed in a variety of tumor cell lines. ROS1 belongs to the "Sevenless" subfamily of tyrosine kinase insulin receptor genes. The protein encoded by this gene is a type 1 integral membrane protein with tyrosine kinase activity. Studies have shown that ROS1 protein may function as a growth or differentiation factor receptor.

One approach for the treatment of a number of hyperproliferative diseases lies in the application of low molecular weight organic substances acting as a kinase inhibitor. An example for such a substance is sunitinib.

Sunitinib, also known as SU11248 is an indolin-2-one compound, which is the active ingredient of the therapeutic agent Sutent® is an orally administered, multi-targeted receptor tyrosine kinase inhibitor for the treatment of renal cell carcinoma (RCC) and imatinib-resistant gastrointestinal stromal tumor (GIST). Sunitinib was the first cancer drug simultaneously approved for two different indications and has become a standard of care for both of these cancers. Administration of SU11248 inhibits intracellular signal transmission of multiple receptor tyrosine kinases leading to both reduced tumor vascularization and cancer cell death, and ultimately tumor shrinkage. In some cases, however, administration of SU11248 and related compounds has been less effective. The reason for this observation was not known.

Thus, there is an urgent need for a method which allows it to select, develop or optimize the efficacy of a therapy and/or a medicament in a subject suffering from a hyperproliferative disorder. In particular, there is a need for a method which makes it possible to reliably prognose or determine if a therapy or a medicament may be effective in a specific case.

The present inventors have identified specific polypeptides as novel targets of anti-cancer drugs, particularly of kinase inhibitor such as indolin-2-one compounds. The presence of these polypeptides, particularly the presence of an increased amount and/or activity of these polypeptides is an indicator that a therapy comprising administration of said anti-cancer drugs is effective. It was shown for the first time that these novel targets are expressed differently in individual cancer cell lines. In other words, an increased or decreased expression level of at least one of the novel targets in a type of cancer cells as compared to the expression level in a non-hyperproliferating wild-type or normal cells, e.g. cells of the same type as the cancer cells directly correlates with the susceptibility of the cancer cells to a treatment with a kinase inhibitor. Therefore, these polypeptides or nucleic acids encoding said polypeptides represent excellent prognostic markers if a drug, particularly a kinase inhibitor as defined herein, for example sunitinib, is effective in the treatment of a subject suffering from a hyperproliferative disease.

In a first aspect, the present invention relates to a method of determining if a subject suffering or suspected to suffer from a hyperproliferative disorder is susceptible to treatment with a kinase inhibitor, comprising measuring the expression level and/or activity of (i) a ROS1, BMP2k, NEK9, NME4 and/or TBK1 polypeptide and/or (ii) a ROS1, BMP2k, NEK9, NME4 and/or TBK1 nucleic acid in a sample comprising diseased cells from said subject, wherein an increased ROS1, BMP2k, NEK9, NME4 and/or TBK1 expression level and/or activity is indicative for susceptibility to said treatment.

In a further aspect, the present invention relates to a diagnostic composition or kit comprising a detection reagent for (i) a ROS1, BMP2k, NEK9, NME4 and/or TBK1 polypeptide or (ii) a ROS1, BMP2k, NEK9, NME4 and/or TBK1 nucleic acid for stratifying of subjects suffering or suspected to suffer from a hyperproliferative disorder with regard to their susceptibility to treatment with a kinase inhibitor.

In still a further aspect, the present invention relates to a method of treating a subject suffering or suspected to suffer from a hyperproliferative disorder with a kinase inhibitor comprising measuring the expression level and/or activity of (i) a ROS1, BMP2k, NEK9, NME4 and/or TBK1 polypeptide and/or (ii) a ROS1, BMP2k, NME4 and/or TBK1 NEK9 nucleic acid in a sample comprising diseased cells from said subject, wherein an increased ROS1, BMP2k, NEK9, NME4 and/or TBK1 expression level and/or activity is indicative for susceptibility to said treatment, selecting a susceptible subject, and administering to said subject a pharmaceutically effective amount of said kinase inhibitor.

In still a further aspect, the present invention relates to a method of treating a subject suffering from a hyperproliferative disorder associated with, accompanied by or caused by an increased expression level of ROS1, BMP2k, NEK9, NME4 and/or TBK1, comprising administering to said subject a pharmaceutically effective amount of a kinase inhibitor.

The target polypeptides of the invention are selected from ROS1, particularly human ROS1, as defined in SEQ ID NO:1, BMP2k, particularly human BMP2k, as defined in SEQ ID NO:2 or 3, NEK9, particularly human NEK9, as defined in SEQ ID NO:4, NME4, particularly human NME4, as defined in SEQ ID NO:5 or TBK1, particularly human TBK1, as defined in SEQ ID NO:6, allelic variants and homologs thereof having at least 70-80%, preferably at least 80-90%, more preferably at least 90-95%, and most preferably at least 95-99.9% or more identity with the sequences of SEQ ID NO:1, 2, 3, 4, 5 or 6 over the entire length, provided that said allelic variants and homologs exhibit the characteristics of a target polypeptide as defined above. Mostly preferred, the target polypeptide is ROS1 as defined in SEQ ID NO:1.

The target nucleic acids encode a target polypeptide selected from ROS1 (SEQ ID NO:1), BMP2k (SEQ ID NO:2 or 3), NEK9 (SEQ ID NO:4), NME4 (SEQ ID NO:5) or TBK1 (SEQ ID NO:6) allelic variants and homologs thereof as defined above. More preferably, the target nucleic acid is a ROS1 nucleic acid having the sequence of SEQ ID NO:7. Further, the target nucleic acid may be selected from a BMP2k nucleic acid having the sequence of SEQ ID NO:8 or 9 and a NEK9 nucleic acid having the sequence of SEQ ID NO:10, a NME4 nucleic acid having the sequence of SEQ ID NO:11 or a TBK1 nucleic acid having the sequence of SEQ ID NO:11.

The target polypeptides or nucleic acids of the invention may be over- or under-expressed in a variety of hyperproliferating (e.g. tumor) cell types as compared to the corresponding non-hyperproliferating wild-type cells. Compared to an expression level observed in wildtype cells of a given cell type or tissue, over- or under-expression means a degree of polypeptide synthesis leading to an amount of said polypeptide within or at the surface of the hyperproliferating cell, which differs from that of the wild-type or normal cell by at least factor 1.5, preferably at least factor 2, more preferably by at least factor 5.

It was shown by the present inventors that the expression profile of the target structures according to the invention may be tumor-type specific. Many of the investigated cancer cell lines exhibit an over-expression of a target structure as defined above. For example, ROS1 is highly expressed in NSCLC cell lines, glioblastoma, pancreas cancer and metastatic renal cancer carcinoma (mRCC), whereas ROS1 is rarely expressed in certain cancer cells of breast and colon tissue.

Further, the activity of the target polypeptide according to the invention, e.g. the enzymatic activity, may be increased or decreased in a variety of hyperproliferating (e.g. tumor) cell types as compared to that of the corresponding non-hyperproliferating wild-type cells. Compared to an activity level observed in wildtype cells of a given cell type or tissue, an increased or decreased enzymatic activity means an alteration in the activity by at least factor 1.5, preferably by at least factor 2, more preferably by at least factor 5.

The targets as defined above can be used for the prognosis if a kinase inhibitor and in particular a tyrosine kinase inhibitor, would be antiproliferatively and/or pro-apoptotically effective and thus suitable for the treatment of a subject suffering from a diagnosed hyperproliferative disease as, for example, a cancer disease, in a given case.

In some embodiments, the invention comprises the step of detecting and/or measuring the expression and/or the activity level of at least one target molecule as defined above in a sample comprising diseased cells from said subject, wherein, e.g. as compared to a sample of non-diseased wildtype cells, an increased expression and/or activity level is indicative for susceptibility to a treatment with a kinase inhibitor.

The subject may be an animal, and in particular-a mammal. In a preferred embodiment, the subject is a human being. In one embodiment, the subject is a cancer patient, e.g. a human cancer patient. In some cases, the subject is a patient who has undergone surgery and/or radiation therapy.

The term "hyperproliferative disorder" or "hyperproliferative disease" refers to conditions associated with, accompanied by or caused by the occurrence of cellular hyperproliferation, particularly associated with invasiveness, migration and/or metastasis formation. More particularly, a hyperproliferative disorder is associated with metastasis formation. A hyperproliferative disorder may be associated with an increased or decreased expression of at least one target molecule as described herein. Further, a hyperproliferative disorder may be associated with an increased or decreased activity, e.g. a kinase activity, particularly a tyrosine kinase activity of at least one target molecule as described herein.

Further, the hyperproliferative disorder may be cancer, e.g. an invasive, migratory and/or metastatic cancer or a non-metastatic cancer. Particularly, the cancer is metastatic cancer. The cancer may be bone, brain, breast, cervix, colon, gastric, kidney, liver, lung, ovary, pancreas, prostate, skin cancer, and more preferably brain cancer, e.g. glioblastoma or lung cancer, e.g. non-small cell lung cancer (NSCLC), gastric cancer, e.g. GIST or renal cancer, e.g. RCC.

According to the present invention it is determined if a subject suffering from a hyperproliferative disorder is susceptible to a treatment with a kinase inhibitor. More preferably, the kinase inhibitor is a tyrosine kinase inhibitor, e.g. an indolin-2-one compound, such as sunitinib.

The expression level and/or activity of a target molecule as defined above may be determined by any appropriate method known to a person skilled in the art.

The expression level and/or activity of a target molecule may be determined on protein level or on the nucleic acid level. For example, a determination of the expression level and/or activity of a target molecule may comprise the steps of
(i) providing sample comprising diseased cells or tissue from a subject suffering or suspected to suffer from a hyperproliferative disease, wherein the sample may be a tissue sample or a body fluid sample,
(ii) contacting the cell sample of step (i) with a detection reagent for a target molecule as described above, and determining the expression level and/or activity of the target molecule, and
(iii) optionally comparing the result with the result from a control sample, e.g. from a sample of non-diseased cells or tissue.

The test sample may be a tissue sample, e.g. a biopsy or a tissue section, or a body fluid sample, e.g. blood, serum, plasma, urine, lymph fluid, cerebrospinal fluid, saliva etc.

For determining the expression level of a target polypeptide, the sample may be subjected to an immunoassay, wherein the detection reagent is used which has the ability to specifically bind to the target polypeptide. For example, the detection reagent may comprise an antibody, an antibody fragment or an aptamer directed against ROS1, BMP2k, NEK9, NME4 or TBK1. Quantitative or semiquantitative immunoassays which can be used herein are known in the art. An example for an applicable test format is an enzyme linked immunosorbent assay (ELISA). In some embodiments, the phosphorylation status of the target polypeptide may be determined using antibodies specifically directed against phosphorylated amino acid residues.

Alternatively or additionally the catalytic/enzymatic activity of a target polypeptide, e.g. a tyrosine kinase, may be determined by methods known in the art.

The determination of the target molecule may also be carried out on nucleic acid level. Determining the expression level of a target nucleic acid can be, for example, carried out by a hybridization assay, optionally combined with an amplification step. Suitable hybridization/amplification protocols are known in the art, such as PCR-based protocols. In this case, the detection reagent may comprise a hybridization probe and optionally an amplification primer. For example, the probe may be single- stranded DNA, RNA, PNA or a nucleic acid analogue having a length of at least 10, preferably at least 15 nucleotides, which is sufficiently complementary to at least one strand of the target nucleic acid that may specifically hybridize with the target nucleic acid under stringent conditions as described, for example, in Sambrook et al. (1989) Molecular Cloning. A laboratory manual, second edition, Cold Spring Harbor Laboratory Press. Stringent conditions may, e.g., comprise a washing step of 30 minutes in 0.1 x SSC, buffer, 0.5% SDS at 55°C, 62°C or 68°C.

The reagent may comprise at least one hybridization probe directed to a nucleic acid encoding a ROS1, NEK9, BMP2k, NME4 or TBK1 nucleic acid as described above, e.g. an mRNA or cDNA. Further, the reagent may comprise at least one amplification primer.

As outlined above, the method according to the invention comprises measuring the expression level and/or activity of at least one target molecule. The method also comprises the measurement of the expression level and/or activity of two or more, preferably 2, 3, 4 or 5 target molecules.

With the method according to the invention, it is now possible to provide a reasonable prognosis for the susceptibility of a subject suffering or suspected to suffer from a hyperproliferative disorder to treatment with a kinase inhibitor.

Therefore, using the method according to the invention may avoid application of kinase inhibitors to subjects which are not susceptible or substantially non-susceptible to treatment. Thus, according to the present invention, the efficacy of cancer treatment may be increased and specific treatment protocols for individual patients may be provided.

For detecting or measuring the expression level of a target molecule selected from ROS1, BMP2k, NEK9, NME4 and/or TBK1 polypeptides and/or nucleic acids, a diagnostic composition or kit may be used. Thus, a further aspect of the present invention relates to a diagnostic composition or kit comprising a detection reagent for detecting or measuring at least one target molecule as defined above.

The detection reagent may comprise an agent which specifically binds to said target molecule under defined conditions. For example, the agent may be an anti-ROS1, anti-BMP2k, anti-NEK9, anti-NME4 or anti-TBK1 antibody, or an antigen binding fragment, or an aptamer. Further, an agent may be a hybridization probe directed a ROS1, BMP2k, NEK9, NME4 and/or TBK1 nucleic acid. The agent as described herein may be coupled with an appropriate labelling group, in order to facilitate detection. Examples for suitable labelling groups, such as radioactive groups, fluorescent groups or other labelling groups are known in the art.

The diagnostic composition or kit according to the invention can be used for stratifying of subjects suffering or suspected to suffer from a hyperproliferative and/or metastatic disorder with regard to their susceptibility to a treatment with a kinase inhibitor.

Another aspect of the present invention relates to a method of treating a subject suffering from a hyperproliferative and/or metastatic disease comprising determining if said subject is susceptible to a treatment with a kinase inhibitor, e.g. sunitinib. This method may comprise the steps of:
- measuring the expression level and/or the activity of at least one target molecule as defined above in a test sample comprising diseased cells or tissue from said subject, wherein, an increase in the expression level and/or activity is indicative for susceptibility to said treatment,
- selecting a susceptible subject, and
- administering to said subject a pharmaceutically effective amount of kinase inhibitor.

A further aspect of the present invention relates to a method for treating a hyperproliferative and/or metastatic disease, comprising administering to a subject in need thereof, a therapeutically effective amount of a kinase inhibitor, wherein said disorder associated with, accompanied by or caused by cells showing an increased expression level of at least one target molecule selected from ROS1, BMP2k, NEK9, NME4 and/or TBK1. The kinase inhibitor is preferably an indolin-2-one compound, and in particular sunitinib. The subject is preferably selected for treatment by being diagnosed for an increased target molecule expression level and/or activity before the treatment. In other embodiments, the subject may be monitored during and/or after the treatment with respect to the target molecule expression level and/or activity. The subject is an animal, particularly a mammal such as a human being.

The hyperproliferative disorder may be cancer, e.g. a cancer as described above, particularly a cancer associated with metastatis formation.

The treatment may comprise administration of a kinase inhibitor, e.g. an indolin-2-one compound, such as sunitinib alone or in combination with other medicaments, e.g. antiproliferative agents, i.e. cytostatic, cytotoxic, chemotherapeutic and/or immunotherapeutic agents, e.g. further kinase inhibitors selected from Lapatinib, Gefitinib, Erlotinib, and/or an anti-tumour antibody, such as Herceptin or Erbitux, and combinations thereof.

The kinase inhibitor may also be administered in combination with other types of therapy such as radiation and/or surgery.

The invention will be described in further detail by the following figures and examples.
Figure 1 (A) is a schematic depiction of a method for the identification of target structures molecules in a cell. The compound SU11248 was coupled to Sepharose and contacted with a total cell lysate under in vitro association conditions. Cellular components bound to SU11248 were identified. Figure 1(B) shows the identification of ROS1 as a target polypeptide for SU11248.
Figure 2 shows the results of an ROS1-expression profiling within different cancer cell lines.
Figure 3 shows the effect of siRNA molecules directed against ROS1, BMP2k, NEK9, NME4 and TBK1 mRNA on the viability (A), caspase activity (B1), apoptosis (B2), cell proliferation (C) and cell morphology (D) of different cancer cell lines.
Figure 4 shows the effect of siRNA molecules directed against ROS-1, BMP2k, NME4 and TBK1 mRNA on SU11248 treatment efficacy in different cancer cell lines.

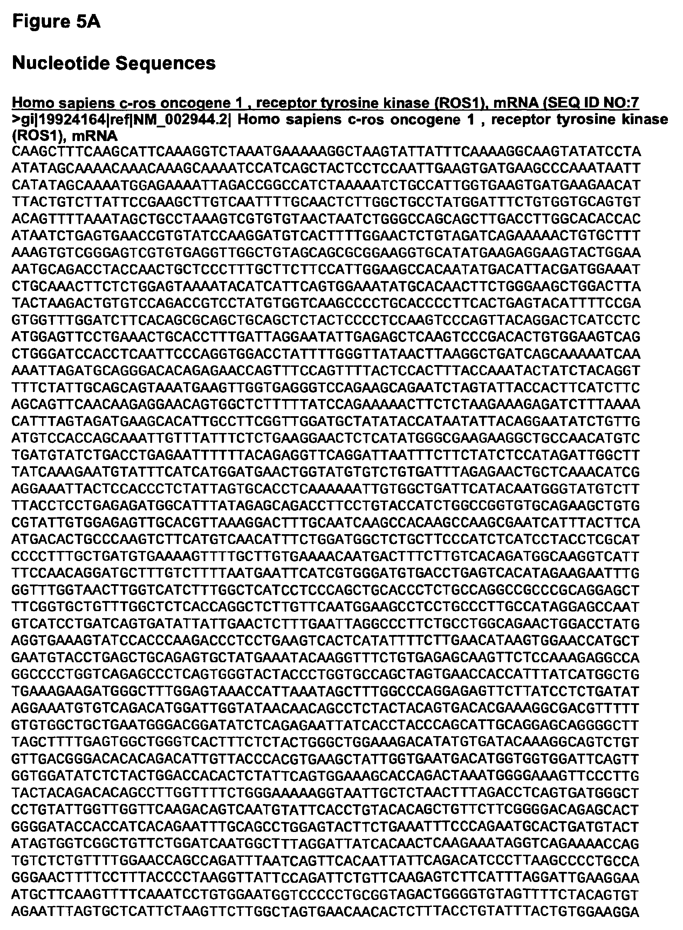

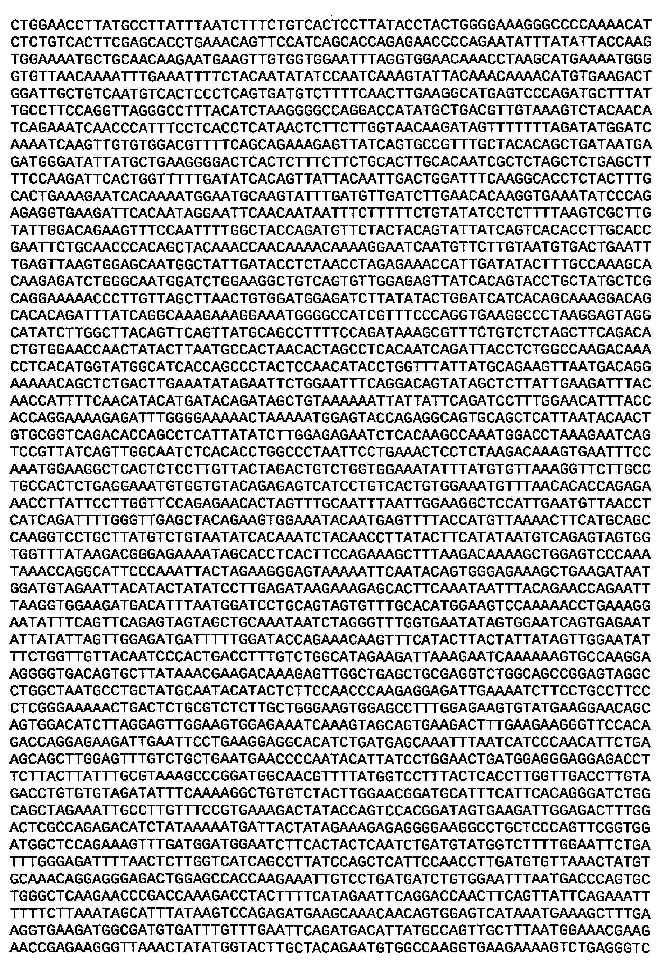

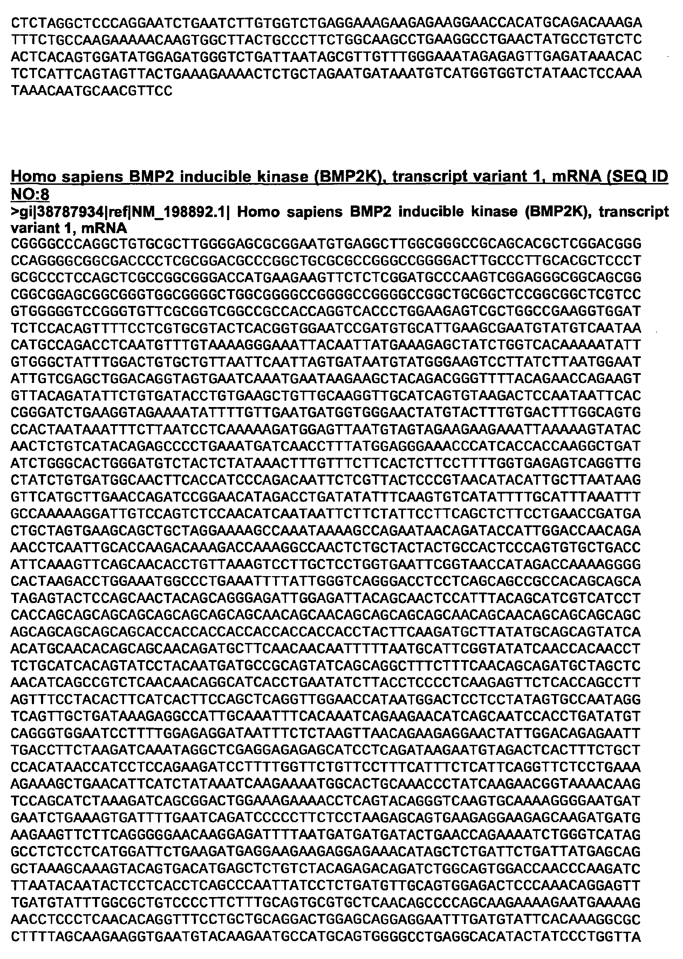

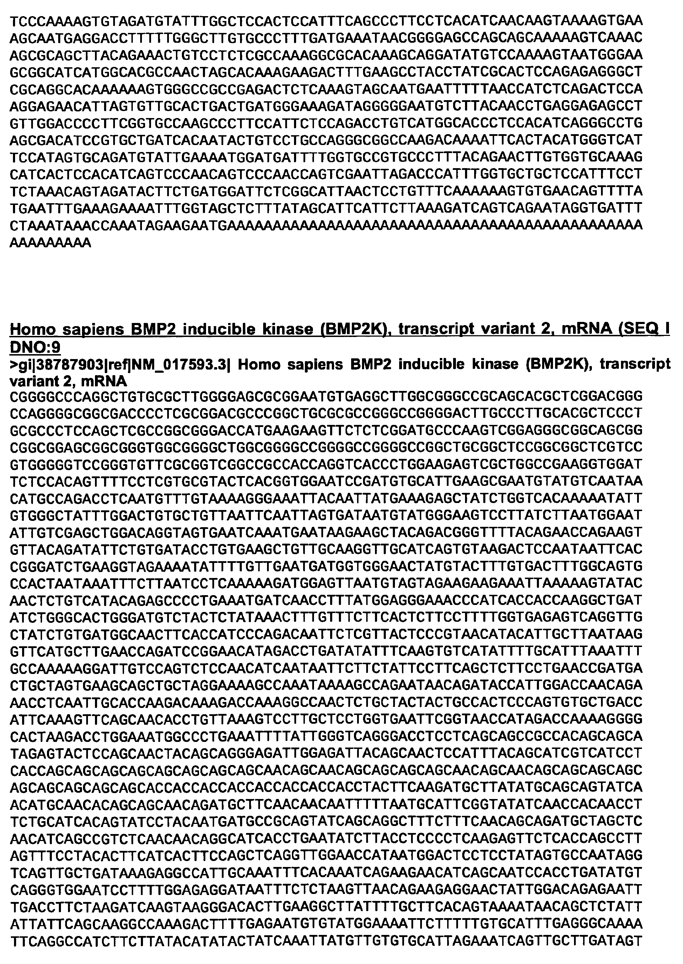

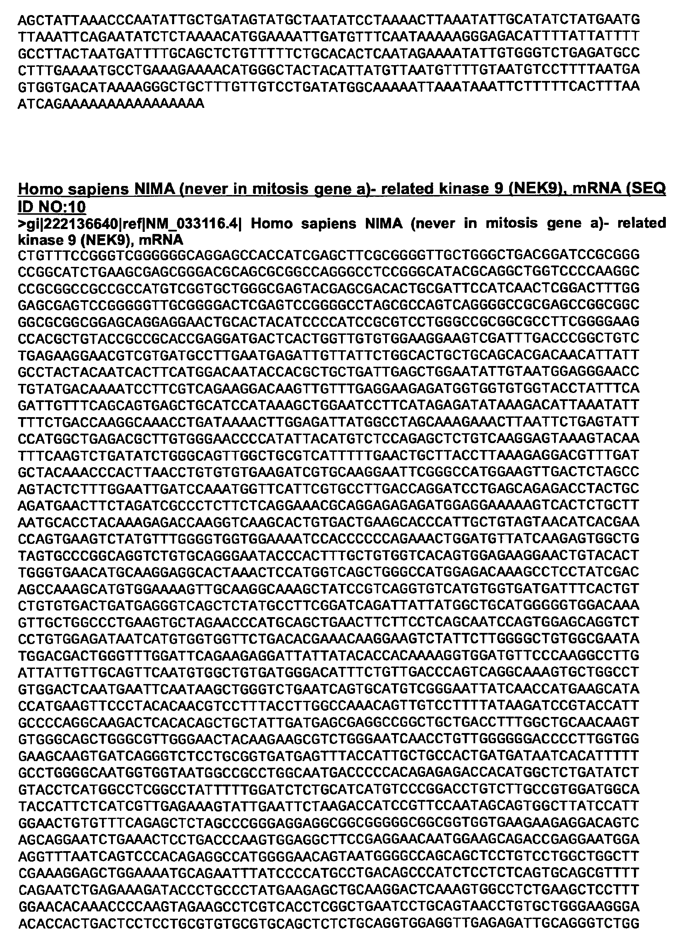

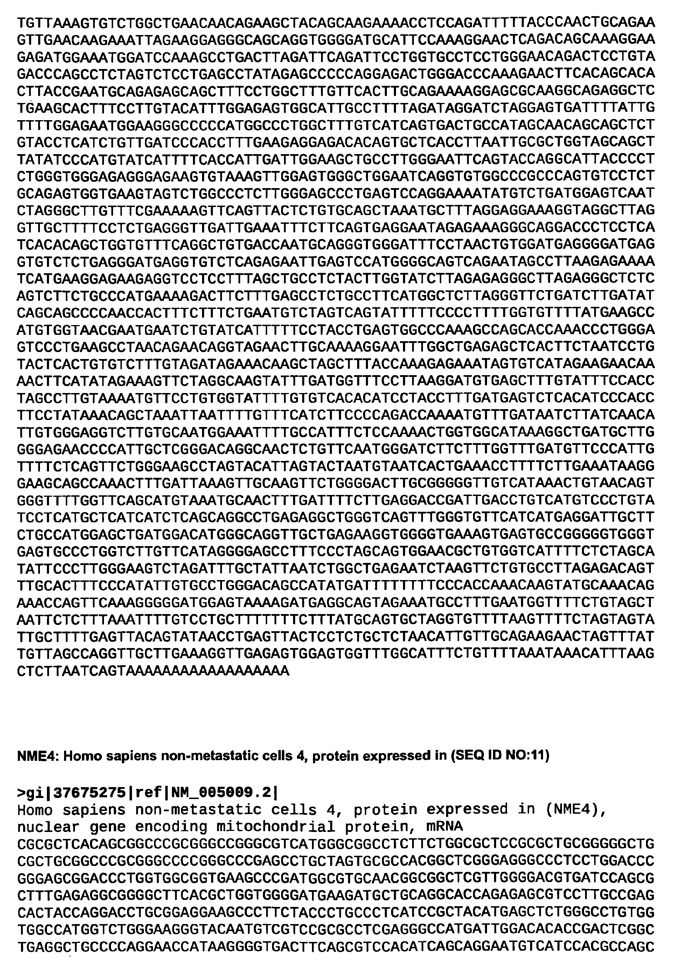

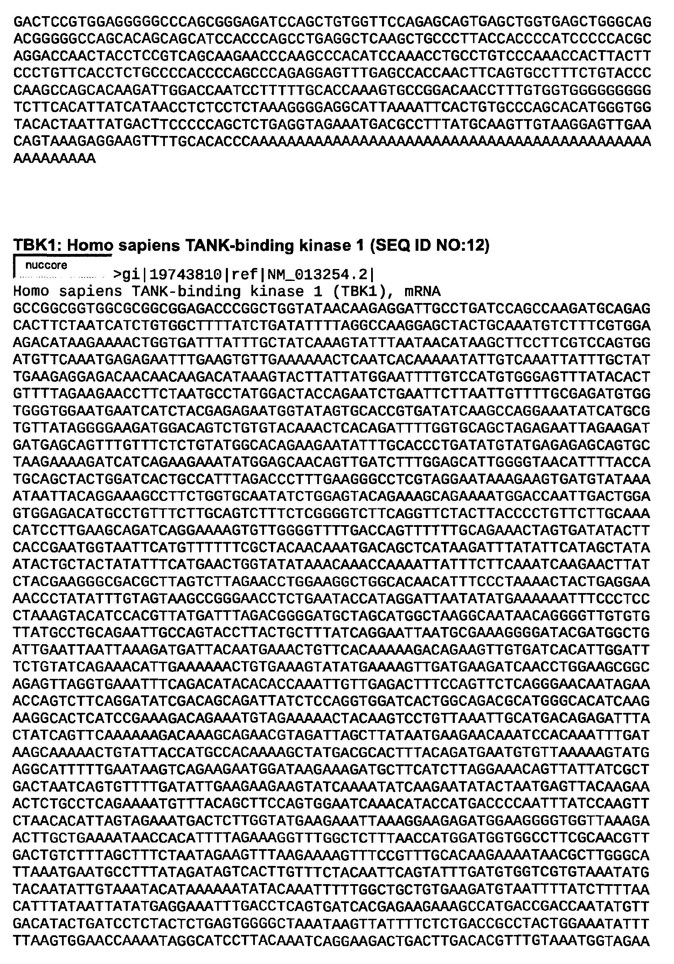

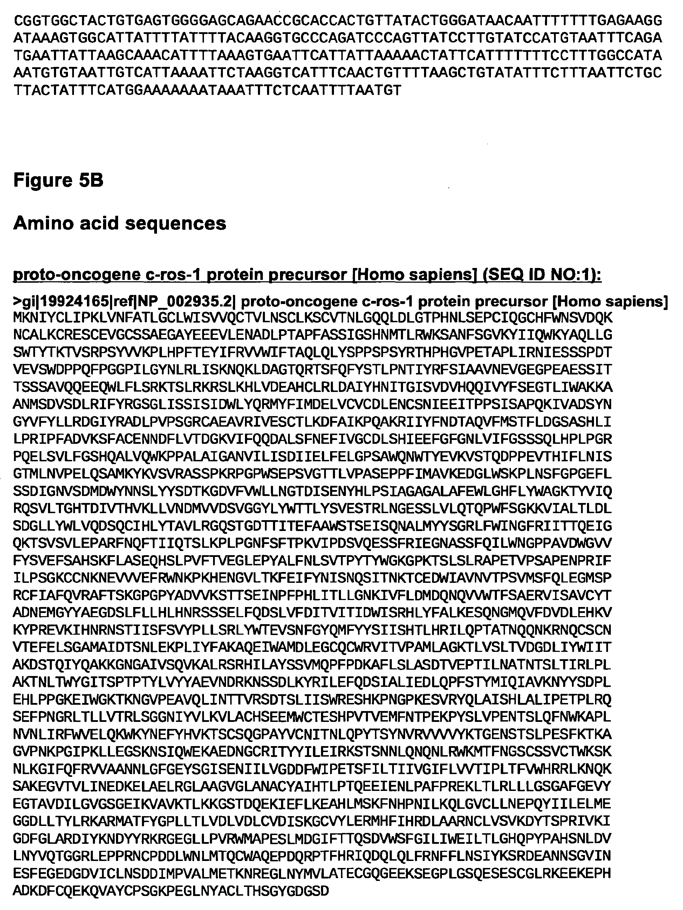

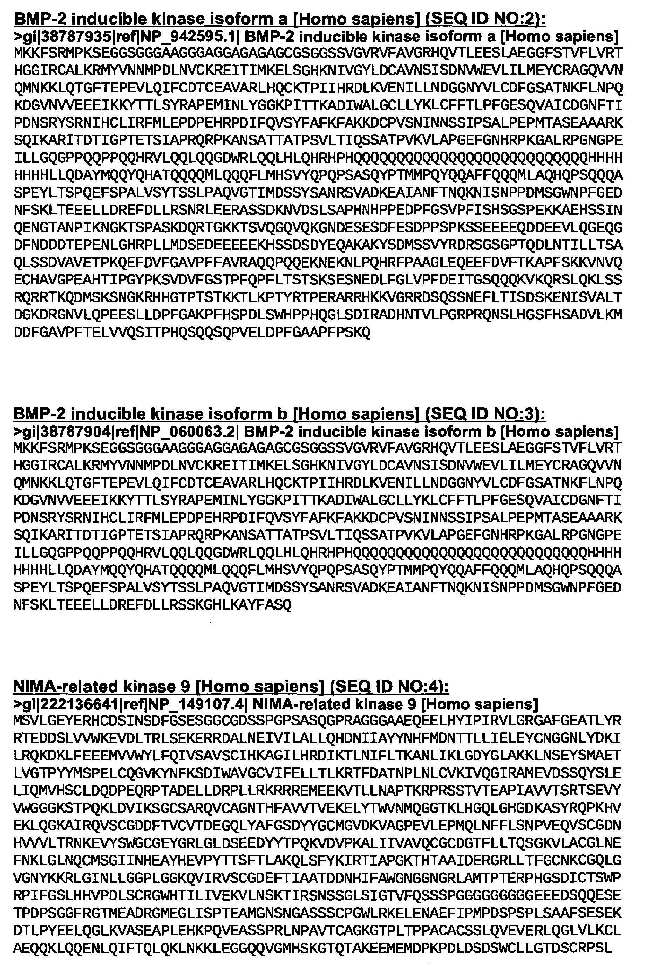

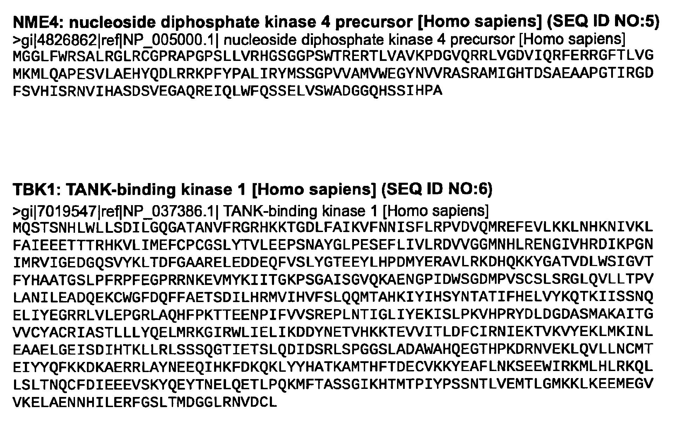

Figure 5 shows the nucleotide (A) and amino acid (B) sequences of ROS1, BMP2k, NEK9, NME4 and TBK1 mRNAs or polypeptides.

### Examples

### 1. Methods

### 1.1 Cellular Assays

### 1.1.1 MTT Assay

In a 96- well flat-bottomed plate, 1000- 2000 cells/100µl cell suspension was seeded. After 24 h, cells were exposed to different concentrations of compound. Each treatment was tested in triplicate wells. At the end of exposure (24 h, 48 h and 72 h), 20 µl of MTT (5 mg/ml in PBS) [3-(4,5-dimethylthiazol-2-y1)-2,5-diphenyltetrazolium bromide; thiazolyl blue, SIGMA, St. Louis, MO] was added to each well, and the plates were incubated at 37 °C for 4 h. Then 50 µl triplex solution (10% SDS, 5% isobutanol, 0.012 M HCI) was added and the plates were incubated at 37°C overnight in a cell incubator. The optical density (OD) was measured using a multiwell spectrophotometer at a wavelength of 570 nm. The inhibitory rate of cell proliferation was calculated by the following formula: Inhibition Rate (%) = [1-(ODtreated- ODtreated (day0)/ODcontrol- ODcontrol (day0))]×100%. The IC₅₀ (i.e. the drug concentration that reduced the absorbance observed in untreated cells by 50%) was calculated by using Hill three parameter log fit in SIGMA Plot 10.

### 1.1.2 SRB Assay

In a 96- well flat-bottomed plate, 1000- 2000 cells/100µl cell suspension was seeded. After 24 h, cells were exposed to different concentrations of compound. Each treatment was tested in triplicate wells. At the end of exposure (24 h, 48 h and 72 h), cells were fixed with ice- cold 10% TCA for 1 h at 4°C, washed with dH₂O and incubated with sulforhodamine B (0.057% in 1% AcCOOH) for 30 min at room temperature. Incorporated dye was dissolved in 10 mM Tris- buffer (pH= 10.5) and the optical density (OD) measured in an ELISA reader at 510 nm. The inhibitory rate of cell proliferation was calculated by the following formula: Inhibition Rate (%) = [1-(ODtreated- ODtreated (day0)/ODcontrol- ODcontrol (day0))]×100%. The IC₅₀ (i.e. the drug concentration that reduced the absorbance observed in untreated cells by 50%) was calculated by using Hill threeparameter log fit in SIGMA Plot 10.0.

### 1.1.3 BrdU Assay

The assay was performed as described in the manufacturer's protocol (Roche). Briefly, in a 96- well flat-bottomed plate, 1000- 2000 cells/100µl cell suspension was seeded. After 24 h, cells were exposed to different concentrations of compound. Each treatment was tested in triplicate wells. At the end of exposure (24 h, 48 h and 72 h) cells were incubated with BrdU for 6- 18 h at 37°C. The labeled cells were fixed with ethanol and prior to incubation with a monoclonal antibody to BrdU, the DNA was partially digested with nucleases to allow the antibody to access BrdU. Next, the anti-BrdU antibody [labeled with peroxidase (POD)] was added for 30 min at 37°C and finally incubated with the POD substrate ABTS for 10- 30 min until coloring of the solution was visible. POD catalyzes the cleavage of ABTS, producing a colored reaction product. The absorbance of the samples was determined with a standard microplate (ELISA) reader at a wavelength of 405 nm.

### 1.1.4 Flow Cytometry

Transfected or compound- treated cells were trypsinized after 72h of siRNA transfection or drug application and collected by centrifugation. For fixation, cells were washed once with PBS, resuspended in 1ml cold 70% ethanol and stored overnight at 4°C. Cells were then collected by centrifugation, washed once with PBS and incubated with 0.01% Triton, 0.1% sodium citrate, 0.02mM propidium iodide (Sigma) in the dark for 2h at 4°C. Cells were analyzed by flow cytometry (FACS Calibur, BD Bioscience). Using the CellQuestPro software, each of the three peaks (representing cells in G₁, S, and G₂/M phases, respectively) obtained in the flow cytometry profile of fluorescence plotted against cell number was gated and quantified.

### 1.1.5 Caspase 3/7- Assay

Caspase 3/7 activity of siRNA transfected or compound- treated cells was measured using the Caspase 3/7 Glo- Assay from Promega according to manufacturer's instruction.

### 1.2 Affinity chromatography and mass spectrometry

### 1.2.1 Compound Synthesis and Immobilization

Sunitinib maleate (SU11248) was purchased from ACC Corporation (San Diego, USA).

For immobilization, SU11248 was chemically modified and kindly provided by VICHEM CHEMIE Ltd., Budapest, Hungary. Drained epoxy-activated Sepharose 6B was resuspended in 2 vol of 0.1, 0.3, 1 or 3 mM Sunitinib dissolved in 50% DMSO/50% 0.05M Na₂CO₃ (pH11). After adding of 10 mM NaOH, coupling was performed overnight at 30°C in the dark. After three washes with 50% DMSO/50% 0.05M Na₂CO₃ (pH11), remaining reactive groups were blocked with 1 M ethanolamine. Subsequent washing steps were performed according to the manufacturer's instructions. To generate the control matrix, epoxy-activated Sepharose 6B was incubated with 1 M ethanol-amine and equally treated as described above. The beads were stored at 4°C in the dark as a suspension in 20% ethanol.

### 1.2.2 Affinity Chromatography

Cells (80 mg protein) were lysed in 30 ml of buffer containing 20 mM Hepes (pH 7.5), 150 mM NaCl, 0.25% Triton X-100, 1 mM EDTA, 1 mM EGTA, 1 mM DTT plus additives (10 mM sodium fluoride/1 mM orthovanadate/10 µg/ml aprotinin/10 µg/ml leupeptin/1 mM phenylmethylsulfonylfluoride/10% glycerol), cleared by centrifugation, and adjusted to 1 M NaCl. The filtrated lysate was loaded with a flow rate of 100 µl/min on an HR 5/2 chromatography column (Amersham Biosciences) containing 600 µl of SU11248 matrix equilibrated to lysis buffer without additives containing 1 M NaCl. The column was washed with 15 column volumes and equilibrated to lysis buffer without additives containing 150 mM NaCl, and bound proteins were eluted in the same buffer containing 1 mM SU11248, 10 mM ATP, and 20 mM MgCl₂ with a flow rate of 50 µl/min. The volume of protein-containing elution fractions was reduced to 1/10 in a Centrivap concentrator (Labonco, Kansas City, MO) before precipitation according to Wessel and Fluegge ((Wessel and Flugge, 1984)). Protein pellets were desolved in 1.5x SDS-sample buffer, pooled and subjected to 1 D- SDS- Gel electrophoresis.

### 1.2.3 1 D-SDS-PAGE and in-gel digestion

Protein pellets after Wessel-Fluegge precipitation were boiled in 1.5 x LDS-sample buffer with 0.5 mM DTT for 10 min at 70°C and subsequently separated by one-dimensional SDS-PAGE using NuPage Novex Bis- Tris gels and NuPage MOPS SDS running buffer (Invitrogen) according to the manufacturer's instruction. The gel was stained with Coomassie Blue using the colloidal blue staining kit (Invitrogen).

Gel bands were cut into 1 mm³ cubes and washed with 50 mM ammonium bicarbonate, 50 % ethanol. For protein reduction, gel pieces were incubated with 10 mM DTT in 50 mM ammonium bicarbonate for 1 h at 56°C. Alkylation of cysteines was performed by incubating the samples with 55 mM iodoacetamide in 50 mM ammonium bicarbonate for 45 min at 25°C in the dark. Gel pieces were washed two times with 50 mM ammonium bicarbonate and incubated with 12.5 ng/µl Trypsin in 50 mM ammonium bicarbonate for 16h at 37°C for protein digestion. Supernatants were transferred to fresh tubes and remaining peptides were extracted by incubating the gel pieces two times in 30% acetonitrile in 3% TFA followed by dehydration with 100% acetonitrile.

The extracts were combined and used for direct peptide identification by mass spectrometry after desalting the samples using RP-C18 stage tip columns.

### 1.2.4 Mass Spectrometric Analysis

All digested peptides were separated by on-line nanoLC and analyzed by tandem mass spectrometry. The experiments were performed on an Agilent 1100 nanoflow system connected to an LTQ-Orbitrap mass spectrometer (Thermo Electron, Bremen, Germany) equipped with a nanoelectrospray ion source (Proxeon Biosystems, Odense, Denmark). The instrument was operated with the "lock mass" option. Binding and separation of the peptides was done in a 15-cm fused silica emitter (75-µm inner diameter from Proxeon Biosystems, Odense, Denmark) in-house packed with reversed-phase ReproSil-Pur C18-AQ 3µm resin (Dr. Maisch GmbH, Ammerbuch-Entringen, Germany).

The peptide mixture was injected onto the column with a flow of 0.5 µl/min and subsequently eluted with a 5-40 % acetonitrile gradient in 0.5 % acetic acid with a flow of 0.25 µl/min. The gradient was 140 min.

Mass spectra were acquired in the positive ion mode applying a data-dependent automatic switch between survey scan and tandem mass spectra (MS/MS) acquisition.

The hybrid linear ion trap/orbitrap instrument was used with the lock mass option in both MS and MS/MS mode. The polydimethylcyclosiloxane (PCM) ions generated in the electrospray process from ambient air were used for internal mass recalibration in real time.

Survey full scan MS spectra (from m/z 300-1800) were acquired in the orbitrap with a resolution r= 60.000 at m/z= 400 after accumulation to a target value of 1.000.000 charges in the linear ion trap. Up to the five most intense ions were sequentially isolated for fragmentation using collisionally induced dissociation at a target value of 30.000 charges. The resulting fragment ions were recorded in the linear ion trap with resolution r=15.000 at m/z=400.

During fragmentation the neutral loss species at 97.97, 48.99 or 32.66 m/z below the precursor ion were activated in turn for 30 ms.

### 1.2.5 Data Analysis

Mass spectrometric data were analyzed with the in-house developed software MaxQuant (version 1.0.12.0 for the proteome and version 1.0.11.5 for the phosphoproteome analysis). MS/MS spectra were searched by Mascot (version 2.2.2, Matrix Science) against the Human IPIBase (version 3.37) combined with common contaminants and concatenated with the reversed versions of all sequences. The following parameters were set for the Mascot searches. Trypsin allowing for cleavage N-terminal to proline and cleavage between aspartic acid and proline was chosen as enzyme specificity. Cysteine carbamidomethylation eas selected as a fixed modification, while protein N-terminal acetylation, methionine oxidation and serine, threonine and tyrosine phosphorylation were selected as variable modifications. Maximally three missed cleavages and up to three labeled amino acids according to SILAC or non- SILAC study were allowed. Initial mass deviation of precursor ion and fragment ions were up to 5 ppm and 0.5 Da, respectively. MaxQuant automatically identified and quantified SILAC peptides and proteins. SILAC protein ratios were calculated as the median of all peptide ratios assigned to the protein. A false discovery rate (FDR) of 0.01 was required for proteins and peptides with a minimum length of 6 amino acids. Furthermore, a posterior error probability (PEP) for each MS/MS spectrum below or equal to 0.01 was required. In case the identified peptides of two proteins were the same or the identified peptides of one protein included all peptides of another protein, these proteins (e.g. isoforms and homologs) were combined by MaxQuant and reported as one protein group.

### 1.3 Molecular methods

### 1.3.1 Cellular kinase assay

A498 kidney cancer cells were seeded at a density of 150.000 cells/well in 6-well flat-bottom cell culture dishes. 24h prior to SU11248 treatment, cells were starved for 24h in medium containing 0% FCS. Drug incubation was performed for 2h, followed by pervanadate stimulation for 5min at 37°C. Cells were lysed and subjected to immunoprecipitation with an antiROS1 antibody over night.

### 1.3.2 RNA interference

Cancer cells were cultured in DMEM, MEM or RPMI medium supplemented with 10 % fetal bovine serum (FBS). 24h prior to RNAi transfection 15.000 cells/ml were seeded into 6-well cell culture plates. At 30% confluency cells were transfected with 30 pmol of validated or pre-designed siRNA from Ambion using RNAiMax (Invitrogen) according to the manufacturer's instruction. GI2 siRNA was taken as control. 5d after transfection cells were used for cell cycle analysis by flow cytometry, MTT-, SRB-, BrdU-assay and western blotting. The knock-down efficiency was monitored by RT-PCR and Western Blotting.

### 1.3.3 Western Blotting

Cells were lysed in RIPA- buffer and equal amounts of protein were resolved by SDS-PAGE. Proteins were transferred to PVDF (Perkin Elmer Polyscreen) membranes, blocked for 1 h in TBS containing 0.1% Tween-20 (TBST) + 4% nonfat dry milk and incubated overnight at 4°C with primary antibody in TBST + 3% BSA. Primary antibodies used included mouse antibodies recognizing pTyrosine (4G10) (1:1000; homemade), rabbit antibodies detecting ROS1 (1:500; Abcam) and a goat antibody for NEK9 (1:1000; Santa Cruz). Membranes were washed three times with TBST and incubated with horseradish peroxidase-conjugated antimouse, antirabbit or antigoat secondary antibody in TBST + 4% nonfat dry milk for 1h at room temperature. Membranes were washed three times with TBST and visualized by ECL (Western Lightning, Perkin Elmer) on X-ray films.

### 2. Results

### 2.1 Identification of target polypeptides for Sunitinib (SU11248)

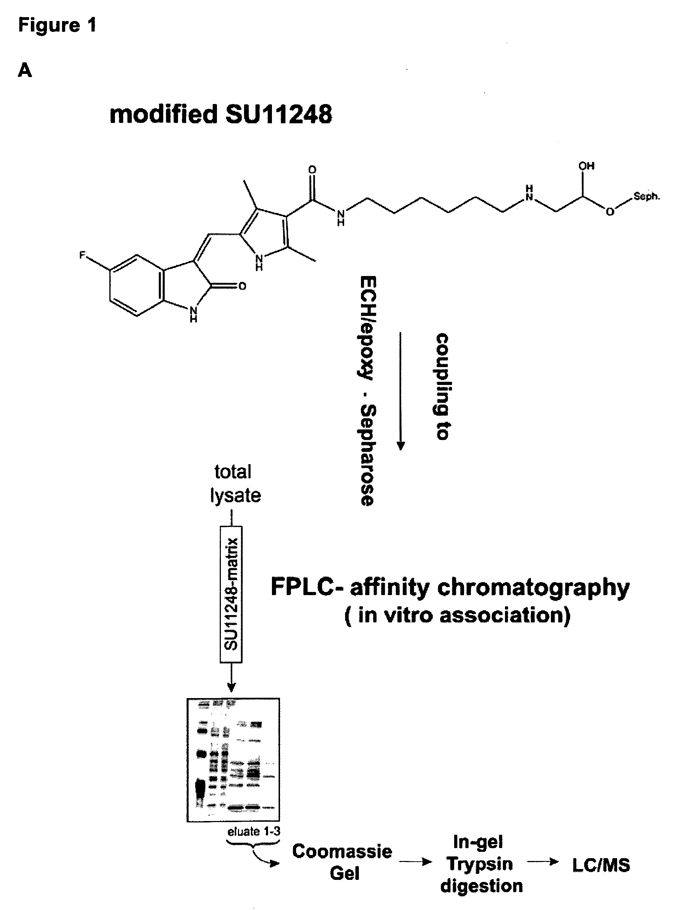

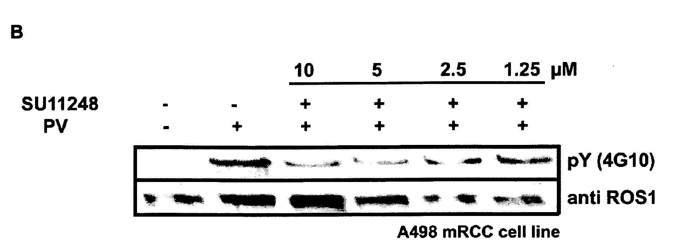

Binding partners of the indolin-2-one compound sunitinib (SU11248) were identified using affinity chromatography and mass spectrometry. For FPLC-affinity chromatography an SU11248 matrix by coupling SU11248 to ECH/epoxy-Sepharose was prepared. Whole cell lysate was incubated with the SU11248 Sepharose matrix under in vitro association conditions. Bound polypeptides were eluted and subjected to LC/MS analysis after in-gel trypsin digestion of PAGE-separated protein bands (Fig. 1A). By this method, the polypeptides ROS1, BMP2k, NEK9, NME4 and TBK1 could be identified as binding partners of SU11248 (cf. Fig. 1 B for ROS1).

### 2.2 Level of ROS1 expression in cancer cells

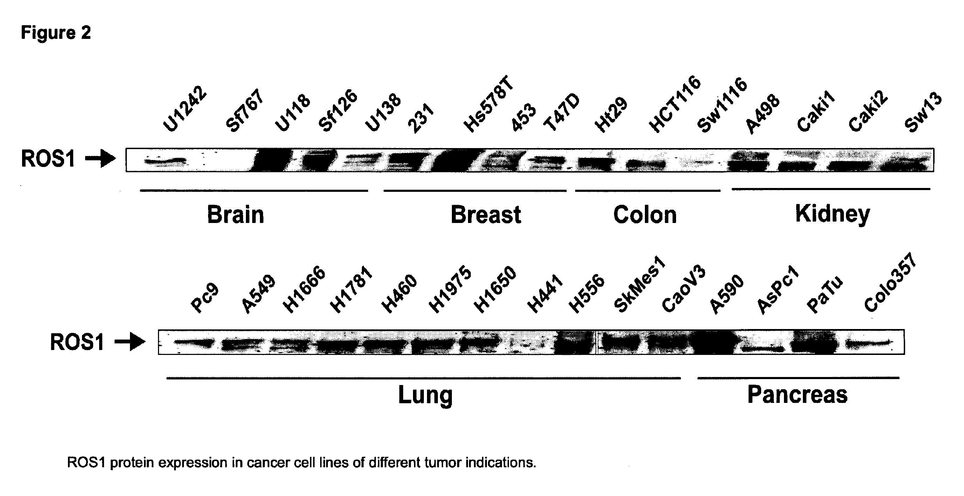

The level of ROS1 expression in several cancer cell lines was tested. The results are shown in Figure 2. It was found that the level of ROS1 expression varies to a large extent in different cancer cells.

### 2.3 Effects of target polypeptide inhibition in cancer cells

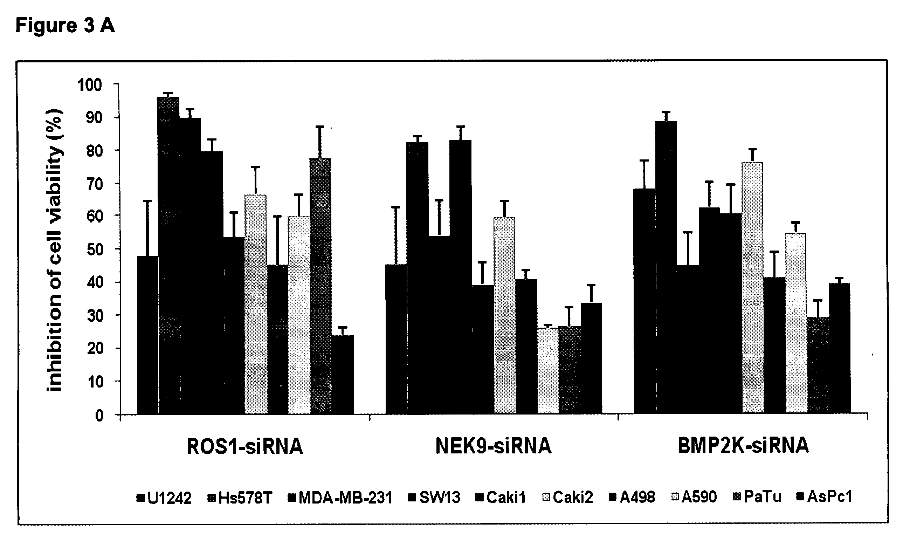

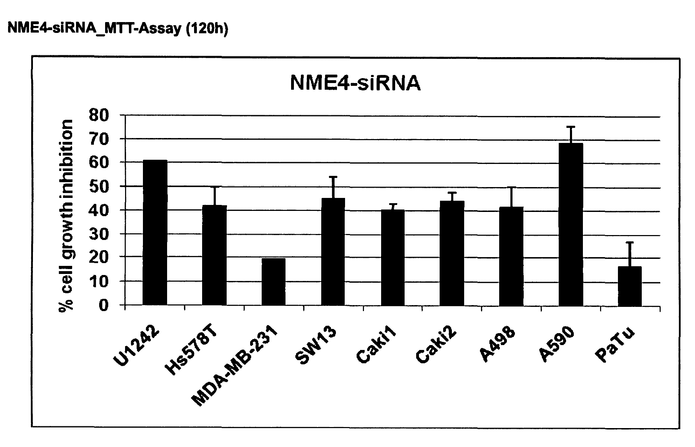

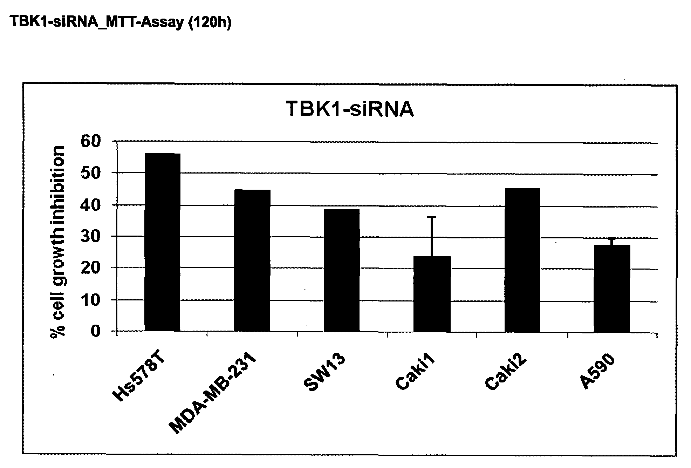

The effect of siRNA molecules specific for ROS1, BMP2k, NEK9, NME4 and TBK1 mRNA on the viability of different cancer cell lines is shown in Fig. 3A (MTT colorimetric assay). In several cell lines a significant inhibition of cell viability was observed.

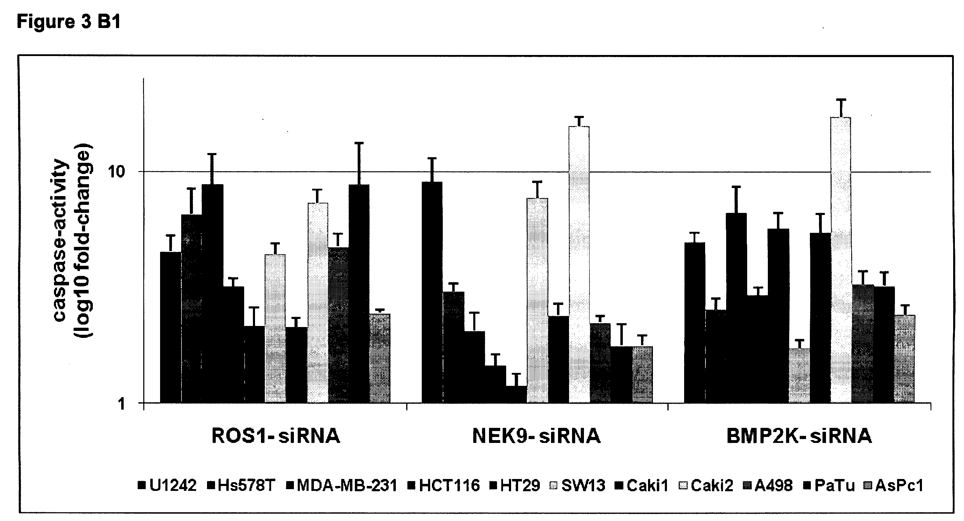

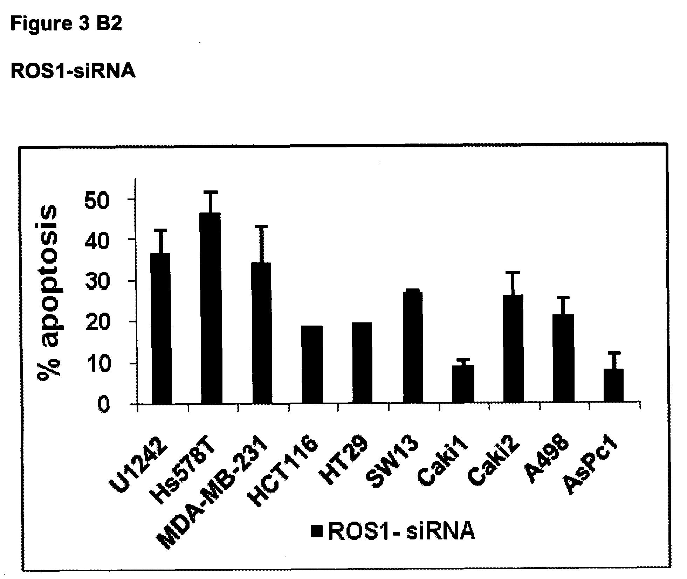

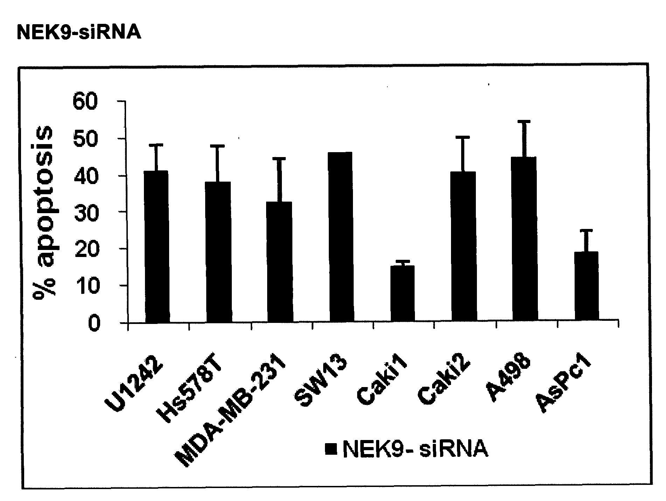

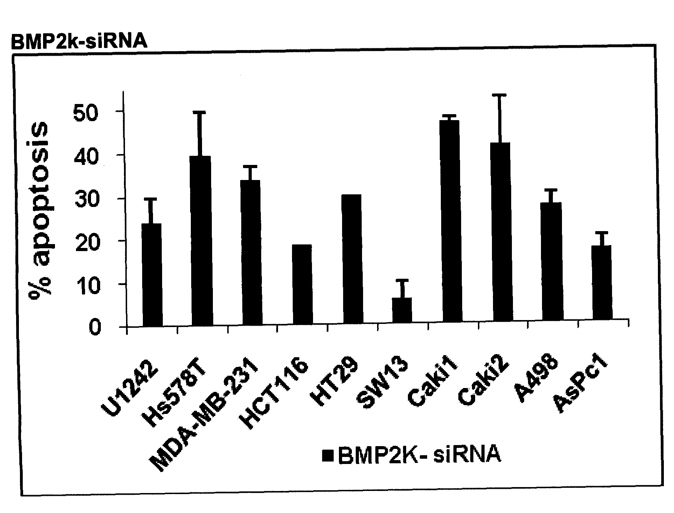

The effect of siRNA molecules specific for ROS1, BMP2k and NEK9 mRNA on the caspase-activity is shown in Fig. 3B1 (caspase assay) and on apoptosis is shown in Fig. 3B2 (FACS-PI assay). In several cancer cell lines an increase of caspase activity and apoptosis was observed.

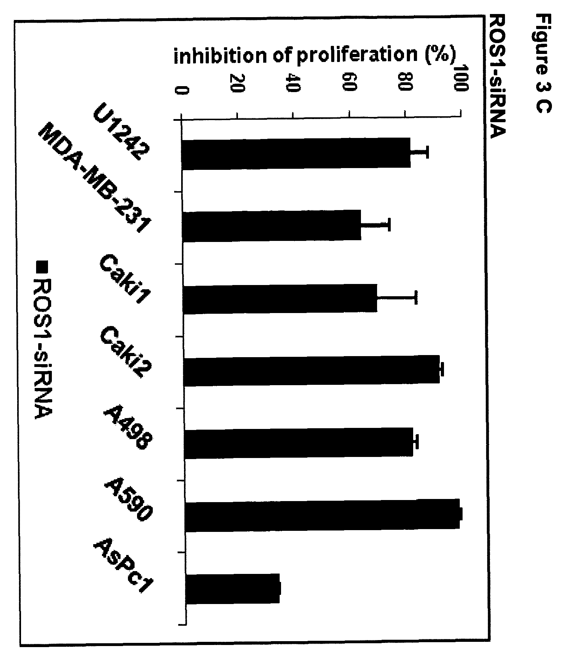

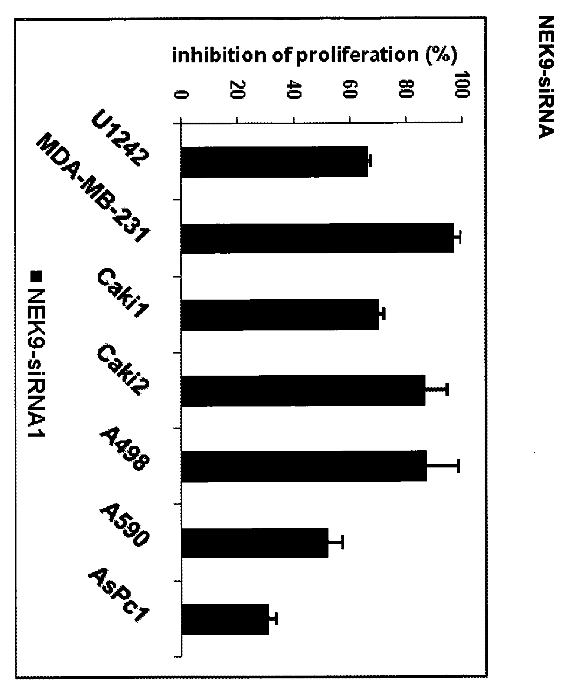

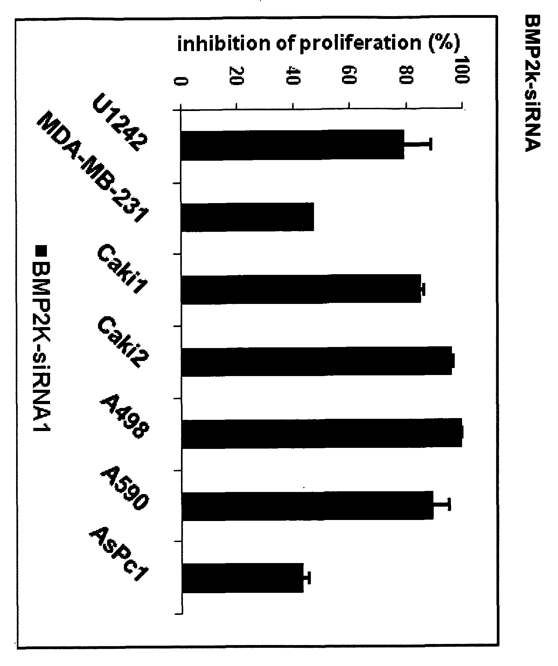

The effect of siRNA molecules specific for ROS1, BMP2k and NEK9 mRNA on the proliferation of different cancer cell lines is shown in Fig. 3C (BrdU-Assay). In several cancer cell lines a significant decrease of cell proliferation was observed.

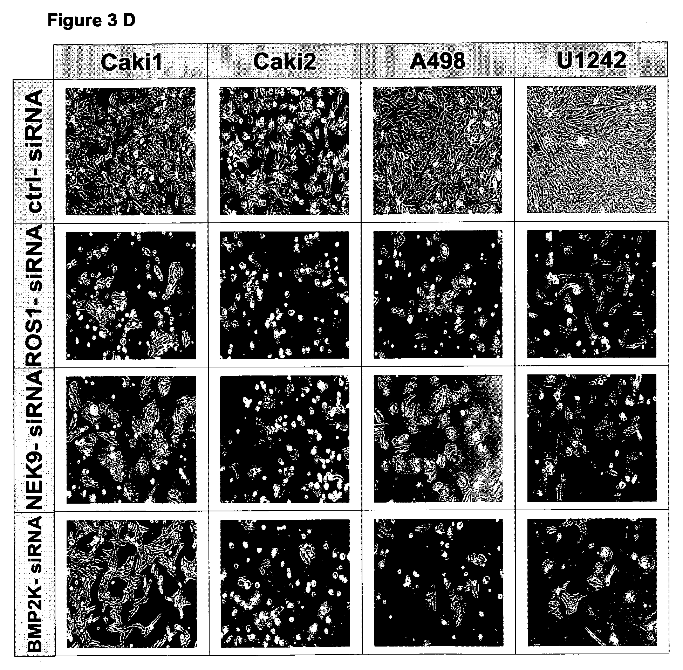

The effect of siRNA specific for ROS1 mRNA, BMP2k mRNA, or NEK9 mRNA versus control (ctrl) siRNA on the morphology of cancer cell lines Caki1, Caki2, A 498 and U1242 is shown in Fig. 3D (microscopic picture). Administration of siRNA leads to morphological changes and a reduction in cell growth.

### 2.4 Effect of target knock-down on efficiency of SU11248 treatment in cancer cells.

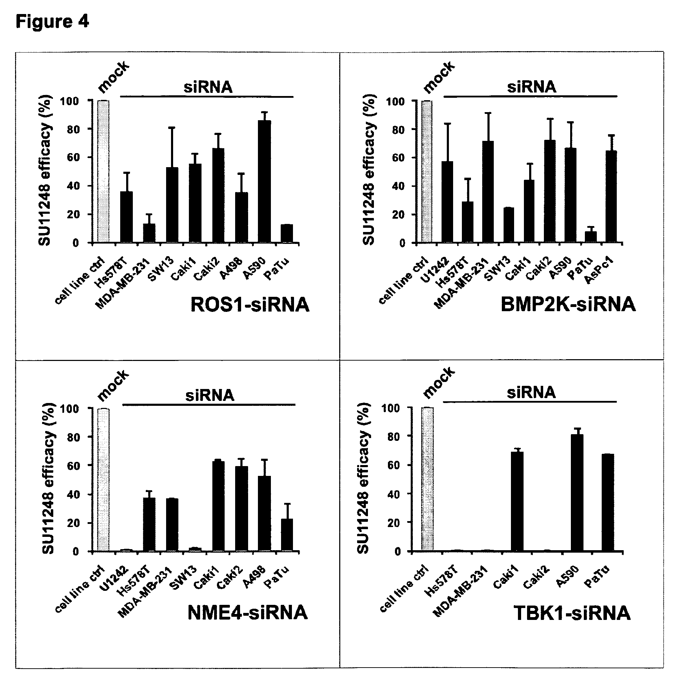

Target depletion was achieved by transient RNAi experiments and the SU11248 efficacy was measured using a standard MTT colorimetric method. Cancer cells were either transfected with control or target-specific siRNA, grown for two days under serum conditions and treated with increasing SU11248 concentrations for 72h. SU11248 efficacy is shown at 5 µM and expressed in percent relative to mock-transfected, SU11248 treated cells. Results were averaged over three to five independent experiments (error bars: SEM). In Fig. 4, it is shown that depletion of ROS1, BMP2k, NME4 and TBK1 by siRNA leads to a dramatic decrease in SU11248 treatment efficacy.

## Claims

1. A method of determining if a subject suffering or suspected to suffer-from a hyperproliferative disorder, particularly from a metastatic disorder, is susceptible to treatment with a kinase inhibitor, comprising measuring the expression level and/or activity of (i) a ROS1, BMP2k, NEK9, NME4 and/or TBK1 polypeptide and/or (ii) a ROS1, BMP2k, NEK9, NME4 and/or TBK1 nucleic acid in a sample comprising diseased cells from said subject, wherein an increased ROS1, BMP2k, NEK9, NME4 and/or TBK1 expression level and/or activity is indicative for susceptibility to said treatment.

2. The method of claim 1, wherein the kinase inhibitor is an indolin-2-one compound, particularly sunitinib.

3. The method of claim 1 or 2, wherein the subject is a cancer patient, particularly a human cancer patient.

4. The method of claim 3, wherein the patient has undergone surgery and/or radiation therapy.

5. The method of claim 3 or 4, wherein the cancer is metastatic or non-metastatic cancer.

6. The method of any one of claims 3 to 5, wherein the cancer is selected from bone, brain, breast, cervix, colon, gastric, kidney, liver, lung, ovary, pancreas, prostate and skin cancer, wherein the cancer is preferably glioblastoma or non-small cell lung cancer.

7. The method of any one of claims 1 to 6, wherein (i) the expression of ROS1, BMP2k, NEK9, NME4 and/or TBK1 is determined on the protein level, e.g. by using an immunoassay and/or by measuring kinase activity, or (ii) the expression of ROS1, BMP2k, NEK9, NME4 and/or TBK1 is determined on the nucleic acid level, e.g. by using a hybridization assay.

8. The method of any one of claims 1 to 7, wherein the sample is a sample comprising cancer cells, e.g. a tissue sample or a body fluid sample.

9. The method of any one of claims 1 to 8, wherein the expression level is increased in said diseased cells at least 1.5-fold, e.g. at least 2-fold, compared to the expression level in normal cells from said subject.

10. A diagnostic composition or kit comprising a detection reagent for (i) a ROS1, BMP2k, NEK9, NME4 and/or TBK1 polypeptide or (ii) a ROS1, BMP2k, NEK9, NME4 and/or TBK1 nucleic acid for stratifying of subjects suffering or suspected to suffer from a hyperproliferative disorder, particularly from a metastatic disorder, with regard to their susceptibility to treatment with a kinase inhibitor.

11. The diagnostic composition of claim 10, wherein the detection reagent comprises (i) an antibody or an antigen binding fragment thereof or (ii) a hybridization probe.

12. A method of treating a subject suffering or suspected to suffer from a hyperproliferative disorder, particularly from a metastatic disorder, with a kinase inhibitor comprising
measuring the expression level and/or activity of (i) a ROS1, BMP2k, NEK9, NME4 and/or TBK1 polypeptide and/or (ii) a ROS1, BMP2k, NME4 and/or TBK1 NEK9 nucleic acid in a sample comprising diseased cells from said subject, wherein an increased ROS1, BMP2k, NEK9, NME4 and/or TBK1 expression level and/or activity is indicative for susceptibility to said treatment,
selecting a susceptible subject, and
administering to said subject a pharmaceutically effective amount of said kinase inhibitor.

13. A method of treating a subject suffering from a hyperproliferative disorder, particularly from a metastatic disorder, associated with, accompanied by or caused by an increased expression level of ROS1, BM-P2k, NEK9, NME4 and/or TBK1, comprising administering to said subject a pharmaceutically effective amount of a kinase inhibitor.

14. The method of claim 12 or 13, wherein the kinase inhibitor is an indolin-2-one compound, particularly sunitinib.

15. The method of any one of claims 12 to 14, wherein the subject is a cancer patient, particularly a human cancer patient.
